(19) Europäisches Patentamt
European Patent Office
Office européen des brevets

(11) Veröffentlichungsnummer: **0 015 387**
B1

# (12) EUROPÄISCHE PATENTSCHRIFT

(45) Veröffentlichungstag der Patentschrift: 12.01.83

(51) Int. Cl.³: C 07 D 249/08, A 01 N 43/64

(21) Anmeldenummer: 80100531.5

(22) Anmeldetag: 04.02.80

(54) **1-Vinyltriazol-Derivate, Verfahren zu ihrer Herstellung sowie ihre Verwendung als Wachstumsregulatoren und Fungizide.**

(30) Priorität: 16.02.79 DE 2906061
22.09.79 DE 2938422

(43) Veröffentlichungstag der Anmeldung: 17.09.80 Patentblatt 80/19

(45) Bekanntmachung des Hinweises auf die Patenterteilung: 12.01.83 Patentblatt 83/2

(84) Benannte Vertragsstaaten: AT BE CH DE FR GB IT NL SE

(56) Entgegenhaltungen:
DE-A-2 645 617
GB-A-2 004 276
US-A-4 182 862

**Die Akte enthält technische Angaben, die nach dem Eingang der Anmeldung eingereicht wurden und die nicht in dieser Patentschrift enthalten sind.**

(73) Patentinhaber: BAYER AG, Zentralbereich Patente, Marken und Lizenzen, D-5090 Leverkusen 1, Bayerwerk (DE)

(72) Erfinder: Reiser, Wolf, Dr., Kiebitzweg 17, D-5600 Wuppertal-1 (DE)
Erfinder: Draber, Wilfried, Dr., In den Birken 81, D-5600 Wuppertal-1 (DE)
Erfinder: Büchel, Karl Heinz, Dr. Prof., Bergerheide 62, D-5600 Wuppertal-1 (DE)
Erfinder: Lürssen, Klaus, Dr., August-Kierspel-Strasse 89, D-5070 Bergisch-Gladbach (DE)
Erfinder: Frohberger, Paul-Ernst, Dr., Willi-Baumeister-Strasse 5, D-5090 Leverkusen (DE)
Erfinder: Paul, Volker, Dr., Ahornstrasse 5, D-5650 Solingen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# 1-Vinyltriazol-Derivate, Verfahren zu ihrer Herstellung sowie ihre Verwendung als Wachstumsregulatoren und Fungizide

Die vorliegende Erfindung betrifft neue 1-Vinyltriazol-Derivate, mehrere Verfahren zu ihrer Herstellung sowie ihre Verwendung als Wachstumsregulatoren und Fungizide.

Es ist bereits bekannt geworden, dass bestimmte 2-Halogen-ethyl-trialkyl-ammonium-halogenide pflanzenwachtsumsregulierende Eigenschaften aufweisen (vergleiche US-Patentschrift 3 156 554). So lässt sich z. B. mit Hilfe von 2-Chlorethyl-trimethyl-ammoniumchlorid eine Beeinflussung des Pflanzenwachstums, insbesondere eine Hemmung des vegetativen Pflanzenwachstums bei wichtigen Kulturpflanzen erzielen. Allerdings ist die Wirksamkeit dieses Stoffes, vor allem bei niedrigen Aufwandmengen, nicht immer ausreichend.

Es ist weiterhin bekannt, dass die 2-Chlorethylphoshonsäure eine wachstumsregulierende Wirkung aufweist (vergleiche DE-OS 1 667 968). Die mit dieser Substanz erzielten Ergebnisse sind jedoch ebenfalls nicht immer zufriedenstellend.

Fernner ist bereits bekannt geworden, dass acylierte und carbamoylierte Derivate von im Phenylteil substituierten 3,3-Dimethyl-1-phenoxy-1-triazolyl-butan-2-olen eine gute fungizide Wirksamkeit besitzen (vergleiche DE-OS 26 00 799). Ebenso eignen sich bestimmte, im Phenylteil substituierte 4,4-Dimethyl-1-phenyl-2-triazolyl-pentan-3-one, wie z. B. 1-(4-Chlorphenyl)-4,4-dimethyl-2-(1,2,4-triazolyl-1-yl)-pentan-3-on, zur Bekämpfung von Pilzen (vergleiche DE-OS 27 34 426). Die Wirkung dieser Azol-Derivate ist jedoch, insbesondere bei niedrigen Aufwandmengen und -konzentrationen, nicht immer ganz befriedigend.

Schliesslich sind pflanzenwuchsregulierende Eigenschaften von solchen Vinyl-triazol-Derivaten bekannt, in denen das Kohlenstoffatom der Vinylgruppe, das den Triazolrest trägt, noch mit einem gegebenenfalls substituierten Benzoylrest verbunden ist und das andere Kohlenstoffatom der olefinischen Doppelbindung mit einem gegebenenfalls substituierten Phenylring verknüpft ist (vgl. DE-OS 26 45 617 und auch ZA-PS 76 - 5920). Die Wirksamkeit dieser Stoffe lässt jedoch in manchen Fällen zu wünschen übrig.

Es wurden nun neue 1-Vinyl-triazol-Derivate der allgemeinen Formel

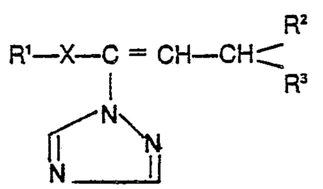

(I)

in welcher

$R^1$ für gegebenenfalls durch Halogen, Alkylcarbonyloxy mit 1 bis 4 Kohlenstoffatomen im Alkylteil, Alkylsulfonyloxy mit 1 bis 4 Kohlenstoffatomen und/oder Phenylsulfonyloxy, welches durch Halogen und/oder Alkyl mit 1 bis 4 Kohlenstoffatomen substituiert sein kann, substituiertes Alkyl mit 1 bis 4 Kohlenstoffatomen, Cycloalkyl mit 5 bis 7 Kohlenstoffatomen sowie Aryl mit 6 bis 10 Kohlenstoffatomen, wobei diese Aryl-Reste substituiert sein können durch Halogen, Alkyl mit 1 bis 4 Kohlenstoffatomen, Phenyl, Phenoxy, Halogenphenyl und/oder Halogenphenoxy, steht,

$R^2$ für Alkyl mit 1 bis 4 Kohlenstoffatomen steht,

$R^3$ für Alkyl mit 1 bis 4 Kohlenstoffatomen, Cycloalkyl mit 5 bis 7 Kohlenstoffatomen, gegebenenfalls durch Alkyl mit 1 bis 4 Kohlenstoffatomen substituiertes Cycloalkenyl mit 5 bis 7 Kohlenstoffatomen, Alkenyl mit 2 bis 4 Kohlenstoffatomen oder für gegebenenfalls durch Halogen und/oder Alkyl mit 1 bis 4 Kohlenstoffatomen substituiertes Aryl mit 6 bis 10 Kohlenstoffatomen steht,

$R^2$ und $R^3$ ausserdem gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen oder Cycloalkyl mit 3 bis 7 Kohlenstoffatomen stehen,

X für die Gruppe $-C(OR^4)R^5-$ sowie zusätzlich für die Ketogruppe steht, wenn $R^1$ für gegebenenfalls substituiertes Alkyl mit 1 bis 4 Kohlenstoffatomen oder Cycloalkyl steht,

$R^4$ für Wasserstoff, Alkyl mit 1 bis 4 Kohlenstoffatomen, gegebenenfalls durch Halogen, Alkyl mit 1 bis 4 Kohlenstoffatomen, Halogenalkyl mit bis zu 2 Kohlenstoffatomen und bis zu 3 gleichen oder verschiedenen Halogenatomen und/oder Phenyl oder Phenoxy, welche ihrerseits durch Halogen substituiert sein können, substituiertes Aralkyl mit 1 oder 2 Kohlenstoffatomen im Alkylteil und 6 bis 10 Kohlenstoffatomen im Arylteil, für den Acylrest $-CO-R^{10}$ oder den Carbamoylrest $-CO-NR^{11}R^{12}$ steht,

$R^5$ für Wasserstoff, Alkyl mit 1 bis 4 Kohlenstoffatomen sowie für gegebenenfalls durch Halogen oder Alkyl mit 1 bis 4 Kohlenstoffatomen substituiertes Aralkyl mit 1 oder 2 Kohlenstoffatomen im Alkylteil und 6 bis 10 Kohlenstoffatomen im Arylteil steht,

$R^{10}$ für Alkyl mit 1 bis 4 Kohlenstoffatomen, Halogenalkyl mit 1 oder 2 Kohlenstoffatomen und 1 bis 5 gleichen oder verschiedenen Halogenatomen sowie für gegebenenfalls durch Halogen und/oder Alkyl mit 1 bis 4 Kohlenstoffatomen substituiertes Phenyl oder Benzyl steht,

$R^{11}$ für Wasserstoff oder Alkyl mit 1 bis 4 Kohlenstoffatomen steht und

$R^{12}$ für Alkyl mit 1 bis 8 Kohlenstoffatomen, Halogenalkyl mit bis zu 4 Kohlenstoffatomen und bis zu 5 gleichen oder verschiedenen Halogenatomen, Halogenalkylmercapto mit bis zu 2 Kohlenstoffatomen und bis zu 5 Halogenatomen, sowie für gegebenenfalls durch Halogen, Alkyl mit 1 bis 4 Kohlenstoffatomen und/oder Halogenalkyl mit bis zu 2 Kohlenstoffatomen und bis zu 5 gleichen oder verschiedenen Halogenatomen substituiertes Aryl mit 6 bis 10 Kohlenstoffatomen steht,

sowie deren Säureadditions-Salze und Metallsalz-Komplexe.

Die Verbindungen der Formel (I) können in zwei geometrischen Isomerenformen vorliegen, je nach Anordnung der Gruppen, die an die Doppelbindung gebunden sind. Wenn X für die Gruppe -C(OR⁴)R⁵- steht, liegt ein asymmetrisches Kohlenstoffatom vor, so dass die Verbindungen der Formel (I) in diesem Fall ausserdem in zwei optischen Isomerenformen anfallen. Die vorliegende Erfindung betrifft sowohl die einzelnen Isomeren als auch Isomeren-Gemische.

Weiterhin wurde gefunden, dass man die 1-Vinyltriazol-Derivate der Formel (I) sowie deren Säureadditions-Salze und Metallsalz-Komplexe erhält, wenn man

a) Triazol-Ketone der Formel

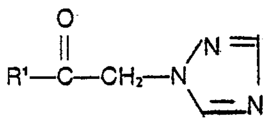

(II)

in welcher

$R^1$ die oben angegebene Bedeutung hat, mit Aldehyden der Formel

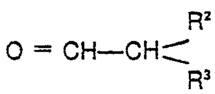

(III)

in welcher

$R^2$ und $R^3$ die oben angegebene Bedeutung haben, in Gegenwart eines Lösungsmittels und in Gegenwart eines Katalysators umsetzt und von den aufgrund der Wasserabspaltung sich bildenden Isomeren das gewünschte isomere Produkt der Formel

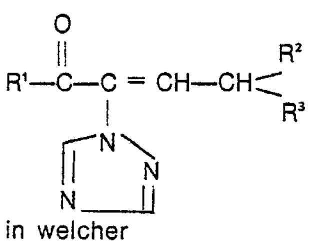

(Ia)

in welcher

$R^1$, $R^2$ und $R^3$ die oben angegebene Bedeutung haben, nach üblichen Methoden isoliert, oder

b) nach dem Verfahren a) erhältliche Verbindungen der Formel

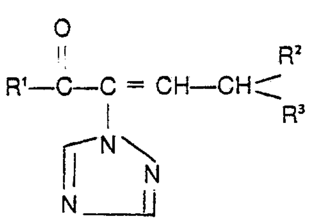

(Ia)

in welcher

$R^1$, $R^2$ und $R^3$ die oben angegebene Bedeutung haben, entweder

α) mit komplexen Hydriden in Gegenwart eines Lösungsmittels reduziert, oder

β) mit Grignard-Verbindungen der Formel

$R^6—Mg—Hal$ (IV)

in welcher

$R^6$ für Alkyl mit 1 bis 4 Kohlenstoffatomen oder für gegebenenfalls durch Halogen oder Alkyl mit 1 bis 4 Kohlenstoffatomen substituiertes Aralkyl mit 1 bis 2 Kohlenstoffatomen im Alkylteil und 6 bis 10 Kohlenstoffatomen im Arylteil steht und

Hal für Chlor, Brom oder Jod steht, in Gegenwart eines Lösungsmittels reduziert, oder

c) nach dem Verfahren (b), Varianten α und β, erhältliche Verbindungen der Formel

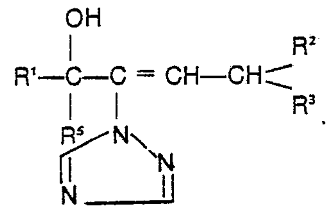

(Ib)

in welcher

$R^1$, $R^2$, $R^3$ und $R^5$ die oben angegebene Bedeutung haben, mit Halogeniden der Formel

$R^7—Hal$ (V)

in welcher

$R^7$ für Alkyl mit 1 bis 4 Kohlenstoffatomen, gegebenenfalls ein- oder mehrfach, gleich oder verschieden durch Halogen, Alkyl mit 1 bis 4 Kohlenstoffatomen, Halogenalkyl mit bis zu 2 Kohlenstoffatomen und bis zu 3 Halogenatomen, gegebenenfalls durch Halogen substituiertes Phenyl oder durch Phenoxy substituiertes Aralkyl mit 1 bis 2 Kohlenstoffatomen im Alkylteil und 6 bis 10 Kohlenstoffatomen im Arylteil, oder für die Reste der Formeln -CO-$R^{10}$ und -CO-$NR^{11}$-$R^{12}$ in welchen $R^{10}$, $R^{11}$ und $R^{12}$ die oben angegebene Bedeutung haben, und

Hal' für Fluor, Chlor oder Brom steht, in Gegenwart eines Lösungsmittels und gegebenenfalls in Gegenwart einer starken Base bzw. gegebenenfalls in Gegenwart eines Säurebindemittels umsetzt, oder

d) nach dem Verfahren (b), Varianten α und β, erhältliche Verbindungen der Formel

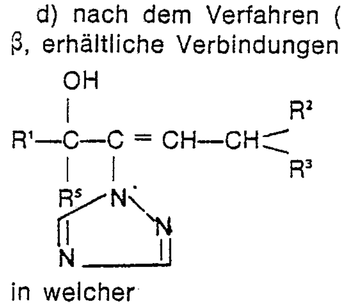

(Ib)

in welcher

$R^1$, $R^2$, $R^3$ und $R^5$ die oben angegebene Bedeutung haben, mit Säureanhydriden der Formel

$R^8—O—R^8$ (VI)

in welcher

$R^8$ für den Acylrest der Formel $-CO-R^{10}$ steht, worin

$R^{10}$ die oben angegebene Bedeutung hat, in Gegenwart eines Lösungsmittels und gegebenenfalls in Gegenwart eines Katalysators umsetzt, oder

e) nach dem Verfahren (b), Varianten α und β, erhältliche Verbindungen der Formel

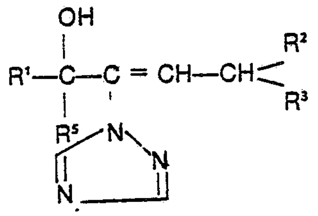

(Ib)

mit Isocyanaten der Formel

$$O = C = N—R^9 \quad \text{(VII)}$$

in welcher

$R^9$ für Alkyl mit 1 bis 8 Kohlenstoffatomen, Halogenalkyl mit bis zu 4 Kohlenstoffatomen und bis zu 5 gleichen oder verschiedenen Halogenatomen oder für gegebenenfalls einfach oder mehrfach gleich oder verschieden durch Halogen, Alkyl mit 1 bis 4 Kohlenstoffatomen, Halogenalkyl mit bis zu 2 Kohlenstoffatomen und bis zu 5 gleichen oder verschiedenen Halogenatomen oder Halogenalkylmercapto mit 1 bis 2 Kohlenstoffatomen und bis zu 5 Halogenatomen substituiertes Aryl mit 6 bis 10 Kohlenstoffatomen steht, in Gegenwart eines Lösungsmittels und gegebenenfalls in Gegenwart eines Katalysators umsetzt, und gegebenenfalls anschliessend an die nach den Verfahren (a) bis (e) erhältlichen 1-Vinyltriazol-Derivate eine Säure oder ein Metall-Salz addiert.

Schliesslich wurde gefunden, dass die neuen 1-Vinyltriazol-Derivate der Formel (I) sowie deren Säureadditions-Salze und Metallsalz-Komplexe starke pflanzenwachstumsregulierende und starke fungizide Eigenschaften besitzen.

Überraschenderweise zeigen die erfindungsgemässen 1-Vinyltriazole sowie deren Säureadditions-Salze und Metallsalz-Komplexe eine bessere pflanzenwachstumsregulierende Wirkung als das bekannte 2-Chlorethyltrimethylammoniumchlorid und als die ebenfalls bekannte 2-Chlorethylphosphonsäure, welche anerkannt gut wirksame Stoffe gleicher Wirkungsart sind. Ausserdem besitzen die erfindungsgemässen Verbindungen überraschenderweise eine bessere fungizide Wirkung, als die aus dem Stand der Technik bekannten acylierten und carbamoylierten Derivate von im Phenylteil substituierten 3,3-Dimethyl-1-phenoxy-1-triazolyl-butan-2-olen und als das ebenfalls bekannte 1-(4-Chlorphenyl)-4,4-dimethyl-2-(1,2,4-triazol-1-yl)-pentan-3-on, die chemisch und wirkungsmässig naheliegende Verbindungen sind. Schliesslich übertreffen die erfindungsgemässen Stoffe bezüglich ihrer pflanzenwuchsregulierenden Wirksamkeit auch solche vorbekannten Vinyl-triazol-Derivate, in denen das Kohlenstoffatom der Vinylgruppe, das den Triazolrest trägt, noch mit einem gegebenenfalls substituierten Benzoylrest verbunden ist, und das andere Kohlenstoffatom der olefinischen Doppelbindung mit einem gegebenenfalls substituierten Phenylring verknüpft ist (vgl. DE-OS 26 45 617 und ZA-PS 76-5920). So eignen sich die erfindungsgemässen Stoffe zum Beispiel wesentlich besser zur Wuchshemmung bei Gerste, Sojabohnen und Baumwolle als das 1-Phenyl-2-(1,2,4-triazol-1-yl)-3-(2,4-dichlorphenyl)-prop-1-en-3-on. Eine derartige Überlegenheit konnte im Hinblick auf den bekannten Stand der Technik nicht vorausgesehen werden.

Die erfindungsgemässen 1-Vinyl-triazole sind durch die Formel (I) allgemein definiert.

Bevorzugt sind diejenigen Verbindungen der Formel (I), in denen $\underline{R^1}$ für tert.-Butyl, Isopropyl, Chlor-tert.-Butyl, Brom-tert.-butyl, Fluor-tert.-Butyl, Acetoxy-tert.-butyl, Methylsulfonyloxy-tert.-butyl, p-Toluolsulfonyloxy-tert.-butyl; 1,3-Dichlor-2-methyl-prop-2-yl; 1,3-Dibrom-2-methyl-prop-2-yl; 1,3-Difluor-2-methyl-prop-2-yl; 1-Chlor-3-brom-2-methyl-prop-2-yl; 1,3-Diacetoxy-2-methyl-prop-2-yl; Cyclohexyl, Phenyl, Chlorphenyl, Bromphenyl, Dichlorphenyl, Fluorphenyl, Methylphenyl, Dimethylphenyl, Chlor-methylphenyl, Biphenylyl, Phenoxyphenyl, Chlorphenylphenyl oder Chlorphenoxyphenyl steht; $\underline{R^2}$ für Methyl, Ethyl, Propyl oder Butyl steht; $\underline{R^3}$ für Methyl, Ethyl, Isopropyl, Cyclohexyl, Cyclohexenyl, Methylcyclohexenyl, Allyl, Methacryl, Phenyl, Chlorphenyl, Dichlorphenyl oder Methylphenyl steht; $\underline{R^2 \text{ und } R^3}$ gemeinsam mit dem Kohlenstoffatom, an das sie gebunden sind, für Cyclopropyl, Cyclobutyl, Cyclopentyl, Cyclohexyl, Cyclohexenyl oder Methylcyclohexenyl stehen; $\underline{X}$ für die Gruppe $-C(OR^4)R^5-$ steht sowie auch für die Ketogruppe steht, wenn $R^1$ für die angegebenen Bedeutungen als gegebenenfalls substituiertes Alkyl oder Cycloalkyl steht; $\underline{R^4}$ für Wasserstoff, Methyl, Ethyl, n-Propyl, Isopropyl, Isobutyl, gegebenenfalls durch Chlor substituiertes Naphthyl, gegebenenfalls einfach oder mehrfach, gleich oder verschieden durch Chlor, Fluor, Methyl, Phenyl, Chlorphenyl, Phenoxy, Chlorphenoxy substituiertes Benzyl, den Acylrest $-CO-R^{10}$ oder den Carbamoylrest $-CO-NR^{11}R^{12}$ steht; $\underline{R^5}$ für Wasserstoff, Methyl, Ethyl, Isopropyl, Benzyl, Chlorbenzyl oder Dichlorbenzyl steht; $\underline{R^{10}}$ für Methyl, Ethyl, Isopropyl, Isobutyl, Chlormethyl, Dichlormethyl oder gegebenenfalls einfach oder mehrfach substituiertes Phenyl und Benzyl mit Chlor, Brom oder Methyl als Substituenten steht; $\underline{R^{11}}$ für Wasserstoff, Methyl oder Ethyl steht und $\underline{R^{12}}$ für Methyl, Ethyl, Chlorethyl, Phenyl, Chlorphenyl, Trifluormethyl-, Chlordifluor-methyl-, Dichlor-fluor-methyl- oder Trichlormethyl-mercapto steht.

Im einzelnen seien ausser den bei den Herstellungsbeispielen genannten Verbindungen folgende Verbindungen der allgemeinen Formel (I) genannt:

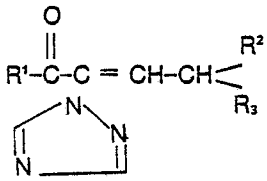

(Ia)

| R¹ | R² | R³ |
|---|---|---|
| $C(CH_3)_3$ | $C_2H_5$ | $C_2H_5$ |
| $C(CH_3)_3$ | $C_2H_5$ | $CH_3$ |
| $C(CH_3)_3$ | $CH_3$ | $CH_3$ |
| $C(CH_3)_3$ | $CH_3$ | 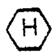 |
| $C(CH_3)_3$ | $CH_3$ | 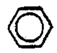 |
| $C(CH_3)_3$ | Cyclopropyl | |
| $C(CH_3)_3$ | Cyclobutyl | |
| $C(CH_3)_3$ | Cyclopentyl | |
| $C(CH_3)_3$ | Cycloheptyl | |
| $C(CH_3)_3$ | Norbon-3-en-2-yl | |
| 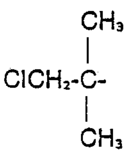 | Cyclohexan | |
| 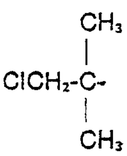 | Cyclohexen | |
| 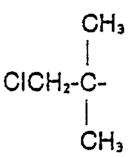 | Methylcyclo-hexen | |
| 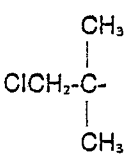 | $CH_3$ | $CH_3$ |
| 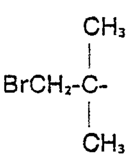 | Cyclohexan | |
| 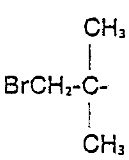 | Cyclohexen | |

| R¹ | R² | R³ |
|---|---|---|
| 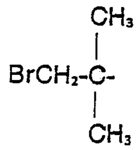 | Methylcyclo-hexen | |
| 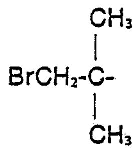 | Methylcyclo-hexen | |
| 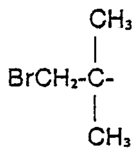 | $CH_3$ | $CH_3$ |
| 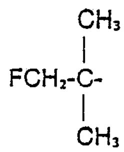 | Cyclohexan | |
| 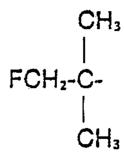 | Cyclohexen | |
| 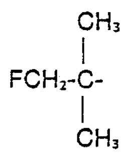 | Methylcyclo-hexen | |
| 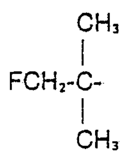 | $CH_3$ | $CH_3$ |
| 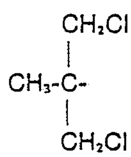 | Cyclohexan | |
| 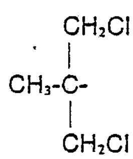 | Cyclohexen | |
| 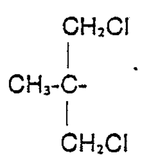 | Methylcyclo-hexen | |
| 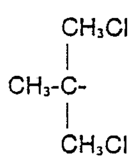 | $CH_3$ | $CH_3$ |

| R¹ | R² | R³ |
|---|---|---|
| 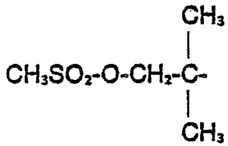 | Cyclohexan | |
| 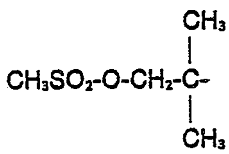 | Cyclohexen | |
| 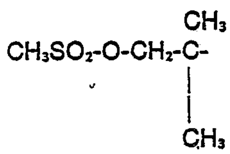 | Methylcyclo-hexen | |
| 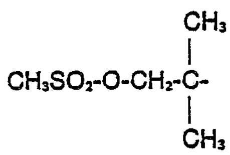 | $CH_3$ | $CH_3$ |
| 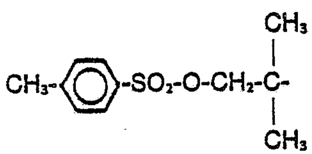 | Cyclohexan | |
| 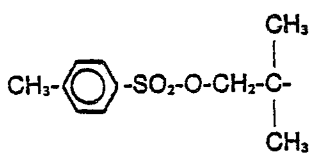 | Cyclohexen | |
| 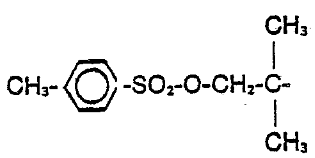 | Methylcyclo-hexen | |
| 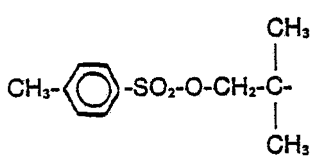 | $CH_3$ | $CH_3$ |
| 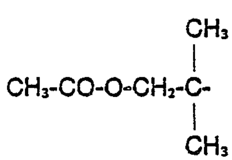 | Cyclohexan | |
| 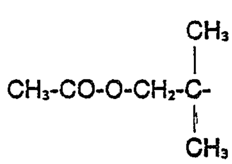 | Cyclohexen | |

| R¹ | R² | R³ |
|---|---|---|
| 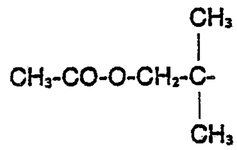 | Methylcyclo-hexen | |
| 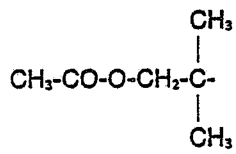 | $CH_3$ | $CH_3$ |
| 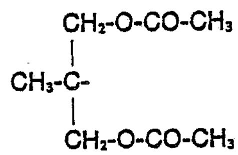 | Cyclohexan | |
| 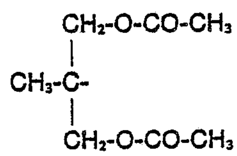 | Cyclohexen | |
| 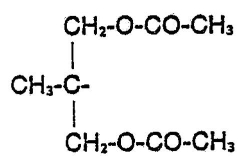 | Methylcyclo-hexen | |
| 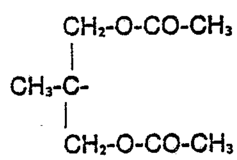 | $CH_3$ | $CH_3$ |
| 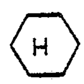 | Cyclohexan | |
| 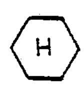 | Cyclohexen | |
| 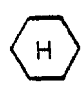 | Methylcyclo-hexen | |
| 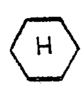 | $CH_3$ | $CH_3$ |

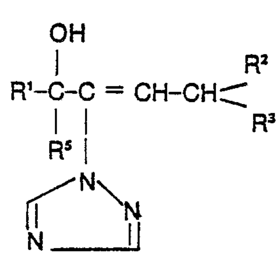

(Ib)

| $R^1$ | $R^2$ | $R^3$ | $R^5$ |
|---|---|---|---|
| $C(CH_3)_3$ | $C_2H_5$ | $CH_3$ | H |
| $C(CH_3)_3$ | $CH_3$ | $CH_3$ | H |
| $C(CH_3)_3$ | $CH_3$ | 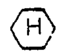 | H |
| $C(CH_3)_3$ | $CH_3$ | 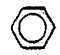 | H |
| $C(CH_3)_3$ | Cyclopropyl | | H |
| $C(CH_3)_3$ | Cyclobutyl | | H |
| $C(CH_3)_3$ | Cyclopentyl | | H |
| $C(CH_3)_3$ | Cycloheptyl | | H |
| $C(CH_3)_3$ | $CH_3$ | $CH_3$ | $CH_3$ |
| $C(CH_3)_3$ | Cyclohexan | | $CH_3$ |
| $C(CH_3)_3$ | Cyclohexen | | $CH_3$ |
| $C(CH_3)_3$ | Methylcyclohexen | | $CH_3$ |
| $C(CH_3)_3$ | $CH_3$ | $CH_3$ | $-CH_2$ 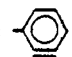 |
| $C(CH_3)_3$ | Cyclohexan | | $-CH_2$ 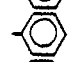 |
| $C(CH_3)_3$ | Cyclohexen | | $-CH_2$ 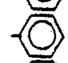 |
| $C(CH_3)_3$ | Methylcyclohexen | | $-CH_2$ 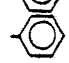 |
| 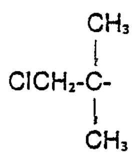 | $CH_3$ | $CH_3$ | H |
| 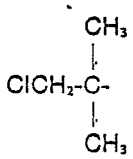 | Cyclohexan | | H |
| 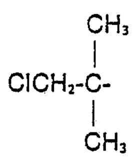 | Cyclohexen | | H |
| 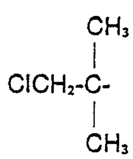 | Methylcyclohexen | | H |
| 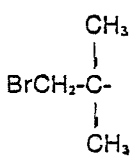 | $CH_3$ | $CH_3$ | H |
| 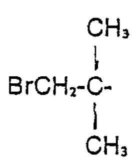 | Cyclohexan | | H |

| $R^1$ | $R^2$ | $R^3$ | $R^5$ |
|---|---|---|---|
| 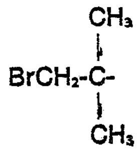 | Cyclohexen | | H |
| 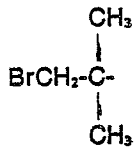 | Methylcyclohexen | | H |
| 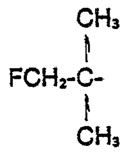 | $CH_3$ | $CH_3$ | H |
| 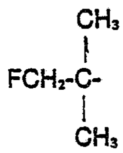 | Cyclohexan | | H |
| 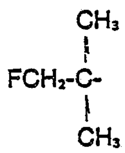 | Cyclohexen | | H |
| 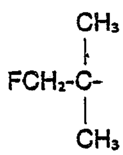 | Methylcyclohexen | | H |
| 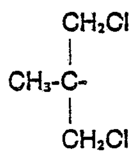 | $CH_3$ | $CH_3$ | H |
| 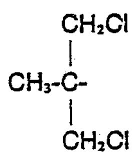 | Cyclohexan | | H |
| 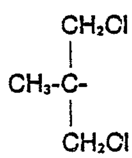 | Cyclohexen | | H |
| 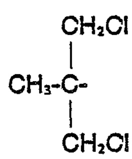 | Methylcyclohexen | | H |
| 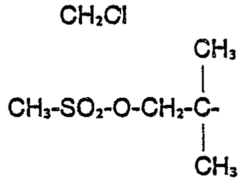 | $CH_3$ | $CH_3$ | H |

| $R^1$ | $R^2$ | $R^3$ | $R^5$ |
|---|---|---|---|
| 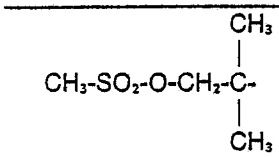 | Cyclohexan | | H |
| 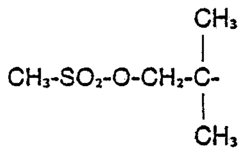 | Cyclohexen | | H |
| 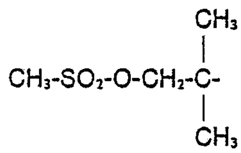 | Methylcyclohexen | | H |
| 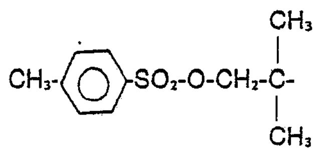 | $CH_3$ | $CH_3$ | H |
| 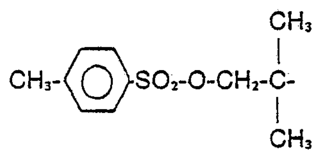 | Cyclohexan | | H |
| 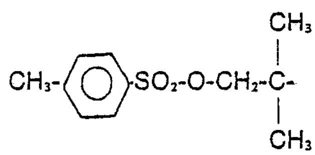 | Cyclohexen | | H |
| 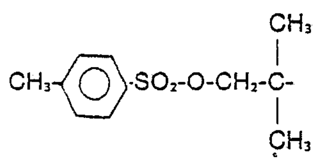 | Methylcyclohexen | | H |
| 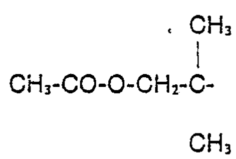 | $CH_3$ | $CH_3$ | H |
| 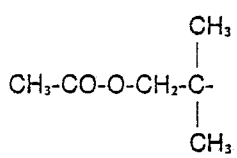 | Cyclohexan | | H |
| 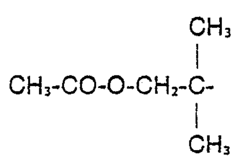 | Cyclohexen | | H |
| 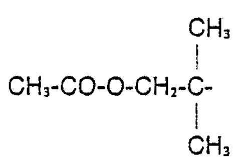 | Methylcyclohexen | | H |

| $R^1$ | $R^2$ | $R^3$ | $R^5$ |
|---|---|---|---|
| 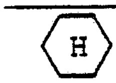 | $CH_3$ | $CH_3$ | H |
| 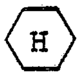 | Cyclohexan | | H |
| 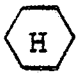 | Cyclohexen | | H |
| 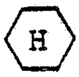 | Methylcyclohexen | | H |
| 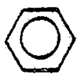 | $CH_3$ | $CH_3$ | H |
| 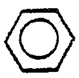 | Cyclohexan | | H |
| 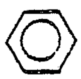 | Cyclohexen | | H |
| 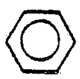 | Methylcyclohexen | | H |
| 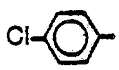 | $CH_3$ | $CH_3$ | H |
| 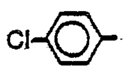 | Cyclohexan | | H |
| 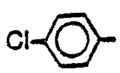 | Cyclohexen | | H |
| 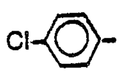 | Methylcyclohexen | | H |
| 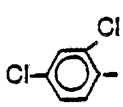 | $CH_3$ | $CH_3$ | H |
| 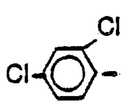 | Cyclohexan | | H |
| 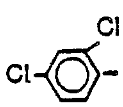 | Cyclohexen | | H |
| 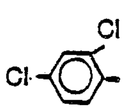 | Methylcyclohexen | | H |

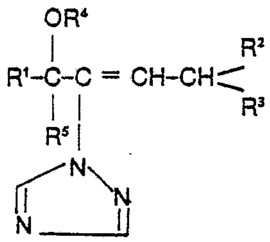

(Ic)

| $R^1$ | $R^2$ | $R^3$ | $R^4$ | $R^5$ |
|---|---|---|---|---|
| $C(CH_3)_3$ | $CH_3$ | $CH_3$ | $C_2H_5$ | H |
| $C(CH_3)_3$ | Cyclohexan | | $C_2H_5$ | H |
| $C(CH_3)_3$ | Cyclohexen | | $C_2H_5$ | H |
| $C(CH_3)_3$ | Methylcyclohexen | | $C_2H_5$ | H |
| 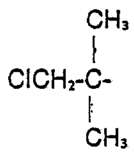 | $CH_3$ | $CH_3$ | $C_2H_5$ | $CH_3$ |
| 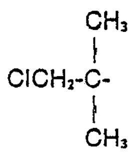 | Cyclohexan | | $C_2H_5$ | $CH_3$ |
| 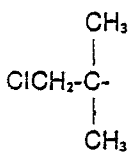 | Cyclohexen | | $C_2H_5$ | $CH_3$ |
| 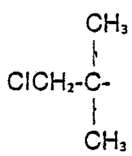 | Methylcyclohexen | | $C_2H_5$ | $CH_3$ |
| 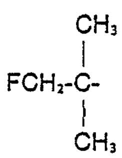 | $CH_3$ | $CH_3$ | $C_2H_5$ | H |
| 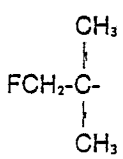 | Cyclohexan | | $C_2H_5$ | H |
| 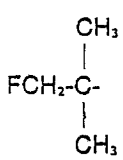 | Cyclohexen | | $C_2H_5$ | H |
| 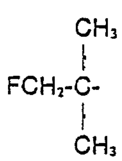 | Methylcyclohexen | | $C_2H_5$ | H |

| $R^1$ | $R^2$ | $R^3$ | $R^4$ | $R^5$ |
|---|---|---|---|---|
| 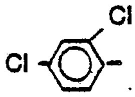 | $CH_3$ | $CH_3$ | $C_2H_5$ | H |
| 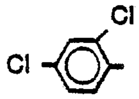 | Cyclohexan | | $C_2H_5$ | H |
| 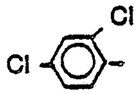 | Cyclohexen | | $C_2H_5$ | H |
| 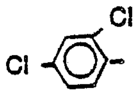 | Methylcyclohexen | | $C_2H_5$ | H |
| $C(CH_3)_3$ | $CH_3$ | $CH_3$ | 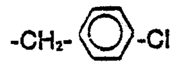 | H |
| $C(CH_3)_3$ | Cyclohexan | | 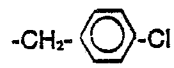 | H |
| $C(CH_3)_3$ | Cyclohexen | | 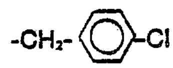 | H |
| $C(CH_3)_3$ | Methylcyclohexen | | 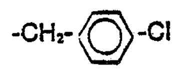 | H |
| 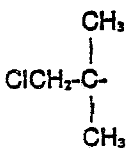 | $CH_3$ | $CH_3$ | 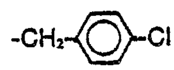 | H |
| 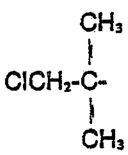 | Cyclohexan | | 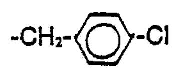 | H |
| 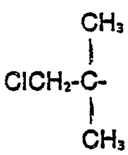 | Cyclohexen | | 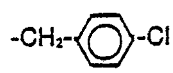 | H |
| 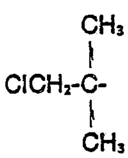 | Methylcyclohexen | | 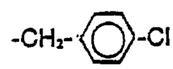 | H |
| 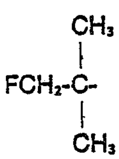 | $CH_3$ | $CH_3$ | 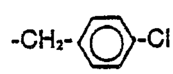 | H |
| 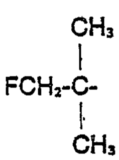 | Cyclohexan | | 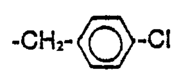 | H |

| $R^1$ | $R^2$ | $R^3$ | $R^4$ | $R^5$ |
|---|---|---|---|---|
| 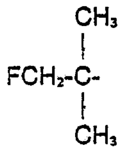 | Cyclohexen | | 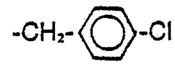 | H |
| 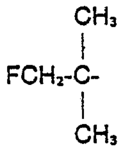 | Methylcyclohexen | | 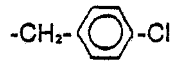 | H |
| 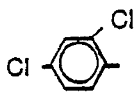 | $CH_3$ | $CH_3$ | 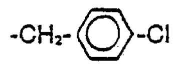 | H |
| 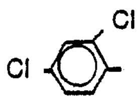 | Cyclohexan | | 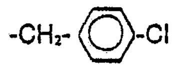 | H |
| 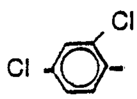 | Cycohexen | | 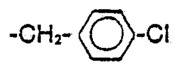 | H |
| 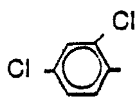 | Methylcyclohexen | | 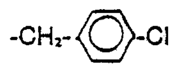 | H |
| 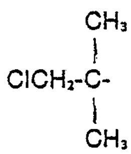 | $CH_3$ | $CH_3$ | $-CO-CH_3$ | H |
| 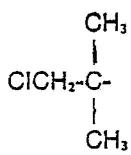 | Cyclohexan | | $-CO-CH_3$ | H |
| 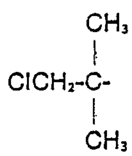 | Cyclohexen | | $-CO-CH_3$ | H |
| 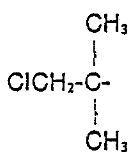 | Methylcyclohexen | | $-CO-CH_3$ | H |
| 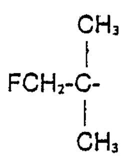 | $CH_3$ | $CH_3$ | $-CO-CH_3$ | H |
| 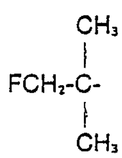 | Cyclohexan | | $-CO-CH_3$ | H |

| $R^1$ | $R^2$ | $R^3$ | $R^4$ | $R^5$ |
|---|---|---|---|---|
| 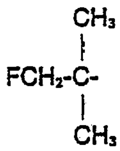 | Cyclohexen | | $-CO-CH_3$ | H |
| 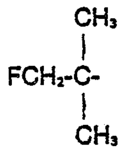 | Methylcyclohexen | | $-CO-CH_3$ | H |
| 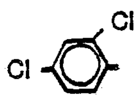 | $CH_3$ | $CH_3$ | $-CO-CH_3$ | H |
| 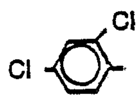 | Cyclohexan | | $-CO-CH_3$ | H |
| 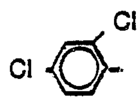 | Cyclohexen | | $-CO-CH_3$ | H |
| 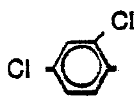 | Methylcyclohexen | | $-CO-CH_3$ | H |
| 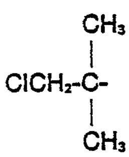 | $CH_3$ | $CH_3$ | $-CO-NHCH_3$ | H |
| 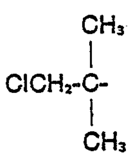 | Cyclohexan | | $-CO-NHCH_3$ | H |
| 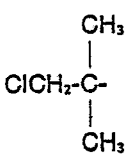 | Cyclohexen | | $-CO-NHCH_3$ | H |
| 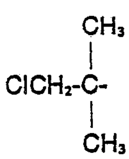 | Methylcyclohexen | | $-CO-NHCH_3$ | H |
| 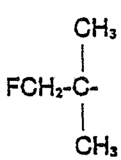 | $CH_3$- | $CH_3$ | $-CO-NHCH_3$ | H |
| 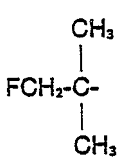 | Cyclohexan | | $-CO-NHCH_3$ | H |

| $R^1$ | $R^2$ | $R^3$ | $R^4$ | $R^5$ |
|---|---|---|---|---|
| 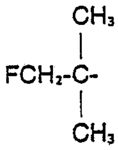 | Cyclohexen | | $-CO-NHCH_3$ | H |
| 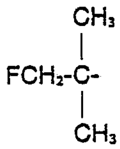 | Methylcyclohexen | | $-CO-NHCH_3$ | H |
| 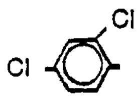 | $CH_3$ | $CH_3$ | $-CO-NHCH_3$ | H |
| 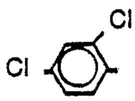 | Cyclohexan | | $-CO-NHCH_3$ | H |
| 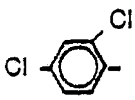 | Cyclohexen | | $-CO-NHCH_3$ | H |
| 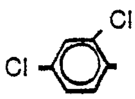 | Methylcyclohexen | | $-CO-NHCH_3$ | H |
| 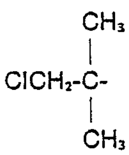 | $CH_3$ | $CH_3$ | -CO-NH- 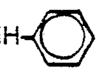 | H |
| 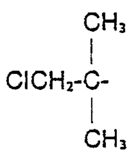 | Cyclohexan | | -CO-NH- 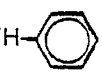 | H |
| 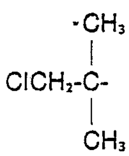 | Cyclohexen | | -CO-NH- 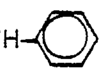 | H |
| 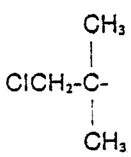 | Methylcyclohexen | | -CO-NH- 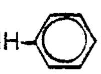 | H |
| 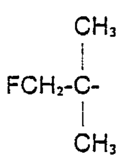 | $CH_3$ | $CH_3$ | -CO-NH- 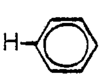 | H |
| 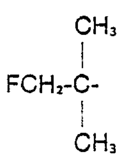 | Cyclohexan | | -CO-NH- 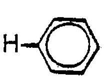 | H |

| R¹ | R² | R³ | R⁴ | R⁵ |
|---|---|---|---|---|
| 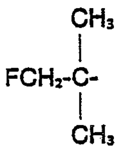 | Cyclohexen | | 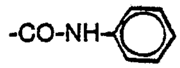 | H |
| 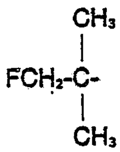 | Methylcyclohexen | | 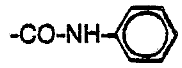 | H |
| 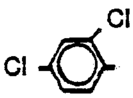 | $CH_3$ | $CH_3$ | 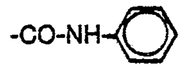 | H |
| 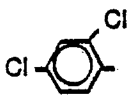 | Cyclohexan | | 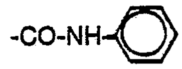 | H |
| 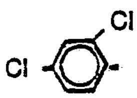 | Cyclohexen | | 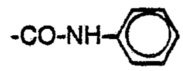 | H |
| 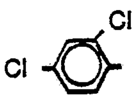 | Methylcyclohexen | | 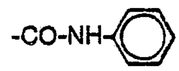 | H |
| 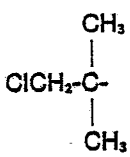 | $CH_3$ | $CH_3$ | 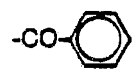 | H |
| 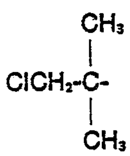 | Cyclohexan | | 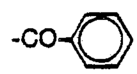 | H |
| 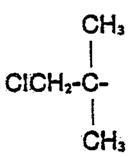 | Cyclohexen | | 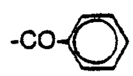 | H |
| 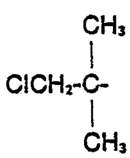 | Methylcyclohexen | | 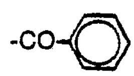 | H |
| 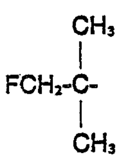 | $CH_3$ | $CH_3$ | 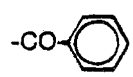 | H |
| 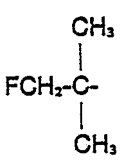 | Cyclohexan | | 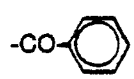 | H |

| R¹ | R² | R³ | R⁴ | R⁵ |
|---|---|---|---|---|
| 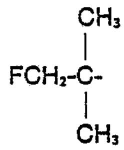 | Cyclohexen | | -CO-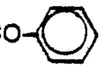 | H |
| 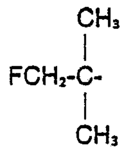 | Methylcyclohexen | | -CO-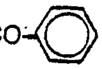 | H |
| 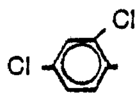 | $CH_3$ | $CH_3$ | -CO-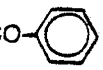 | H |
| 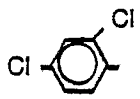 | Cyclohexan | | -CO-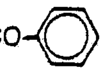 | H |
| 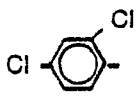 | Cyclohexen | | -CO-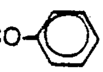 | H |
| 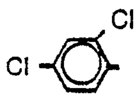 | Methylcyclohexen | | -CO-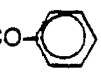 | H |
| 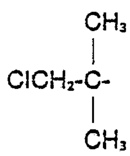 | $CH_3$ | $CH_3$ | $-CO-CHCl_2$ | H |
| 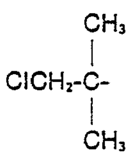 | Cyclohexan | | $-CO-CHCl_2$ | H |
| 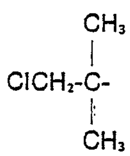 | Cyclohexen | | $-CO-CHCl_2$ | H |
| 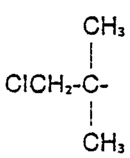 | Methylcyclohexen | | $-CO-CHCl_2$ | H |
| 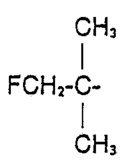 | $CH_3$ | $CH_3$ | $-CO-CHCl_2$ | H |
| 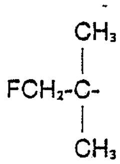 | Cyclohexan | | $-CO-CHCl_2$ | H |

| R¹ | R² | R³ | R⁴ | R⁵ |
|---|---|---|---|---|
| 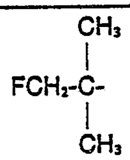 | Cyclohexen | | $-CO-CHCl_2$ | H |
| 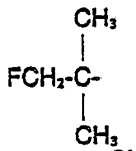 | Methylcyclohexen | | $-CO-CHCl_2$ | H |
| 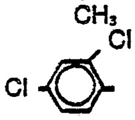 | $CH_3$ | $CH_3$ | $-CO-CHCl_2$ | H |
| 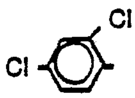 | Cyclohexan | | $-CO-CHCl_2$ | H |
| 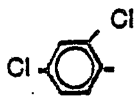 | Cyclohexen | | $-CO-CHCl_2$ | H |
| 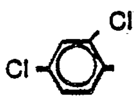 | Methylcyclohexen | | $-CO-CHCl_2$ | H |
| $C(CH_3)_3$ | $CH_3$ | $CH_3$ | $-CO-CH_3$ | H |
| $C(CH_3)_3$ | Cyclohexan | | $-CO-CH_3$ | H |
| $C(CH_3)_3$ | Cyclohexen | | $-CO-CH_3$ | H |
| $C(CH_3)_3$ | Methylcyclohexen | | $-CO-CH_3$ | H |
| $C(CH_3)_3$ | $CH_3$ | $CH_3$ | $-CO-NHCH_3$ | H |
| $C(CH_3)_3$ | Cyclohexan | | $-CO-NHCH_3$ | H |
| $C(CH_3)_3$ | Cyclohexen | | $-CO-NHCH_3$ | H |
| $C(CH_3)_3$ | Methylcyclohexen | | $-CO-NHCH_3$ | H |
| $C(CH_3)_3$ | $CH_3$ | $CH_3$ | -CO-NH- 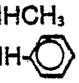 | H |
| $C(CH_3)_3$ | Cyclohexan | | -CO-NH- 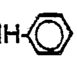 | H |
| $C(CH_3)_3$ | Cyclohexen | | -CO-NH- 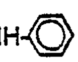 | H |
| $C(CH_3)_3$ | Methylcyclohexen | | -CO-NH- 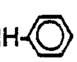 | H |
| $C(CH_3)_3$ | $CH_3$ | $CH_3$ | -CO- 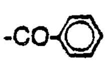 | H |
| $C(CH_3)_3$ | Cyclohexan | | -CO- 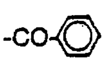 | H |
| $C(CH_3)_3$ | Cyclohexen | | -CO- 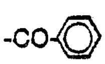 | H |
| $C(CH_3)_3$ | Methylcyclohexen | | -CO- 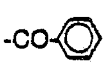 | H |
| $C(CH_3)_3$ | $CH_3$ | $CH_3$ | $-CO-CHCl_2$ | H |
| $C(CH_3)_3$ | Cyclohexan | | $-CO-CHCl_2$ | H |
| $C(CH_3)_3$ | Cyclohexen | | $-CO-CHCl_2$ | H |
| $C(CH_3)_3$ | Methylcyclohexen | | $-CO-CHCl_2$ | H |

Bevorzugte erfindungsgemässe Verbindungen sind auch Additionsprodukte aus Säuren und denjenigen 1-Vinyltriazol-Derivaten der Formel (I), in denen die Reste $R^1$, $R^2$, $R^3$ und X die Bedeutungen haben, die bereits für diese Reste genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Ausserdem bevorzugte erfindungsgemässe Verbindungen sind Additionsprodukte aus Salzen von Metallen der II. bis IV. Haupt- und der I. und II. sowie IIV. bis VIII. Nebengruppe und denjenigen 1-Vinyltriazol-Derivaten der Formel (I), in denen die Reste $R^1$, $R^2$, $R^3$ und X die Bedeutungen haben, die bereits für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäuren und die Bromwasserstoffsäure, ferner Phosphorsäure, Salpetersäure und Schwefelsäure.

Verwendet man beispielsweise Pinakolyl-1,2,4-triazol und Cyclohexancarbaldehyd als Ausgangsstoffe, so kann der Reaktionsablauf nach dem Verfahren (a) durch das folgende Formelschema wiedergegeben werden:

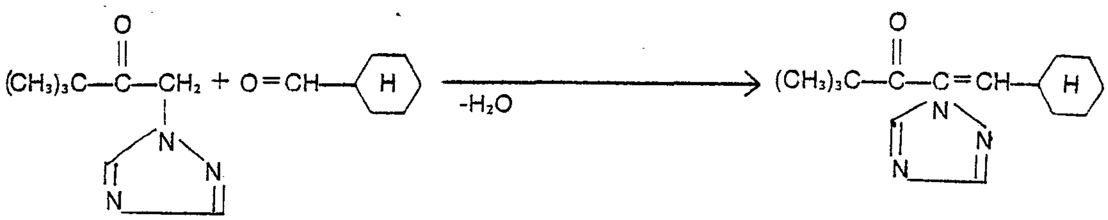


Verwendet man 1-Cyclohexyl-4,4-dimethyl-2-(1,2,4-triazol-1-yl)-pent-1-en-3-on und Natriumborhydrid als Ausgangsstoffe, so kann der Reaktionsablauf nach Variante α des Verfahrens (b) durch das folgende Formelschema wiedergegeben werden:

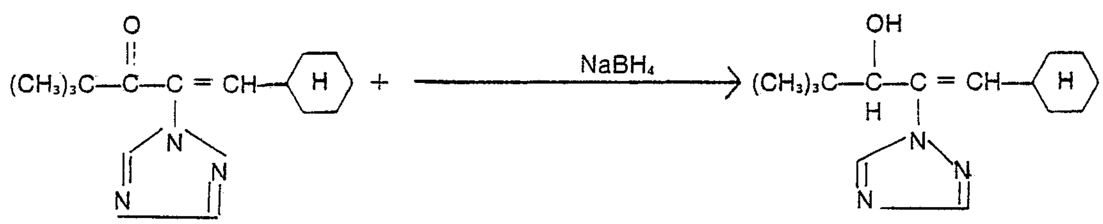


Verwendet man 1-Cyclohexyl-4,4-dimethyl-2-(1,2,4-triazol-1-yl)-pent-1-en-3-on und Methylmagnesiumbromid als Ausgangsstoffe, so kann der Reaktionsablauf nach Variante β des Verfahrens (b) durch das folgende Formelschema wiedergegeben werden:

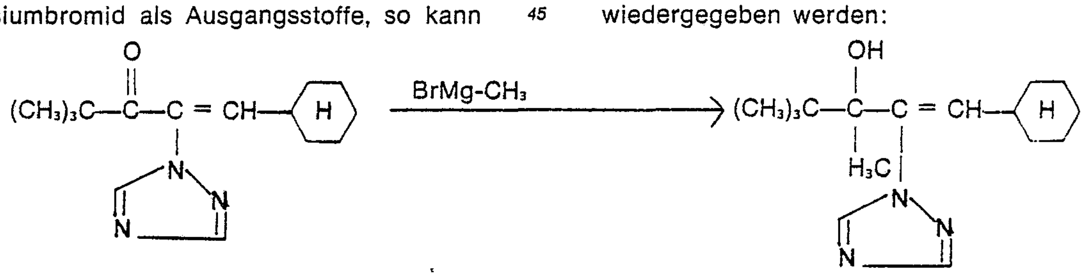


Verwendet man 1-Cyclohexyl-4,4-dimethyl-1-(1,2,4-triazol-1-yl)-pent-1-en-3-ol und Ethylbromid als Ausgangsstoffe und Natriumhydrid als Base, so kann der Reaktionsablauf nach dem Verfahren (c) durch das folgende Formelschema wiedergegeben werden:

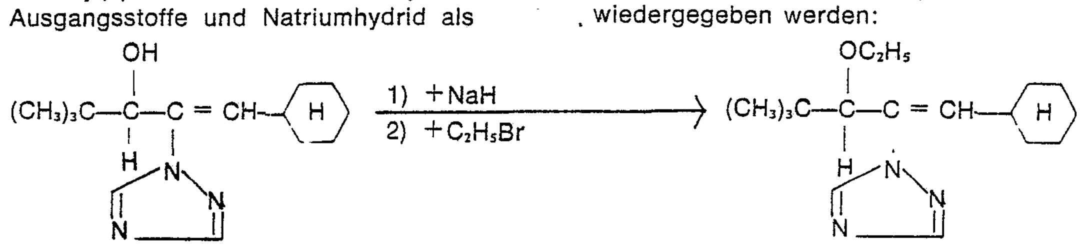

Verwendet man 1-Cyclohexyl-4,4-dimethyl-2-(1,2,4-triazol-1-yl)-pent-1-en-3-ol und Acetylchlorid als Ausgangsstoffe und Natriumhydrid als Base, so kann der Reaktionsablauf nach Verfahren (c) durch das folgende Formelschema wiedergegeben werden:

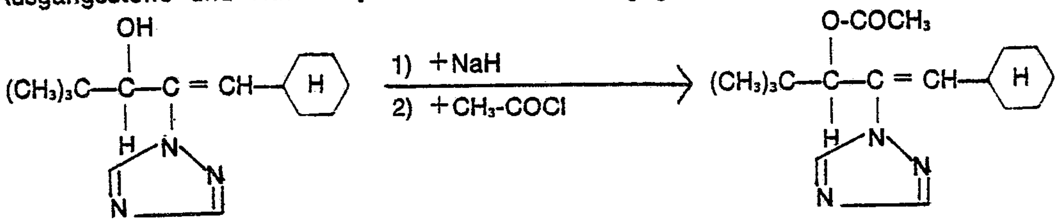


Verwendet man 1-Cyclohexyl-4,4-dimethyl-2-(1,2,4-triazol-1-yl)-pent-1-en-3-ol und Essigsäureanhydrid als Ausgangsstoffe so kann der Reaktionsablauf nach Verfahren (d) durch das folgende Formelschema wiedergegeben werden:

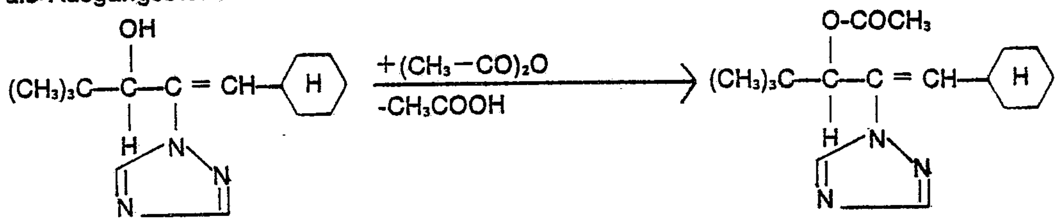


Verwendet man 1-Cyclohexyl-4,4-dimethyl-2-(1,2,4-triazol-1-yl)-pent-1-en-3-ol und Phenylisocyanat als Ausgangsstoffe, so kann der Reaktionsablauf nach Verfahren (e) durch das folgende Formelschema wiedergegeben werden:

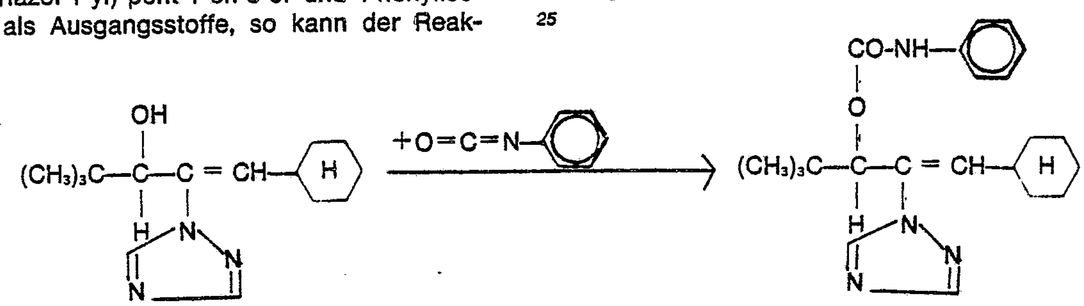

Die bei der Durchführung des erfindungsgemässen Verfahrens (a) als Ausgangsstoffe benötigten Triazol-ketone sind durch die Formel (II) allgemein definiert. In dieser Formel steht $R^1$ für diejenigen Reste, die bereits im Zusammenhang mit der Beschreibung der erfindungsgemässen Stoffe der Formel (I) für diesen Substituenten genannt wurden.

Die Triazol-ketone der Formel (II) sind weitgehend bekannt (vergleiche DE-OS 24 31 407, DE-OS 26 10 022 und DE-OS 26 38 470). Die noch nicht speziell bekannten Verbindungen der Formel (II) lassen sich nach üblichen Methoden herstellen. Sie werden erhalten, indem man die entsprechenden Halogen-ketone in Gegenwart eines Säurebinders mit 1,2,4-Triazol umsetzt. Als Beispiele seien die Verbindungen der folgenden Tabelle genannt:

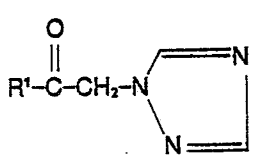

(II)

| $R^1$ | $R^1$ | $R^1$ |
|---|---|---|
| $-C(CH_3)_3$ | $-CH(CH_3)_2$ | $-CH_3$ |
| 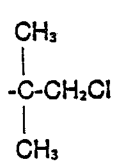 | 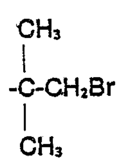 | 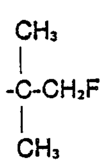 |

| $R^1$ | $R^1$ | $R^1$ |
|---|---|---|
| 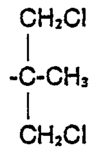 | 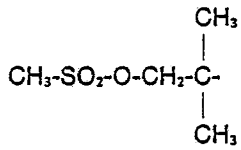 | 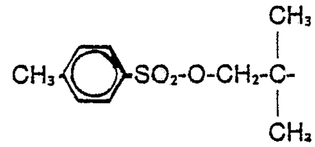 |
| 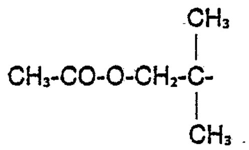 | 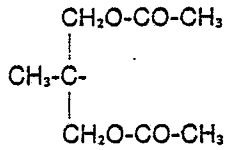 | 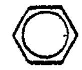 |
| 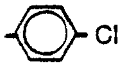 | 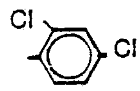 | 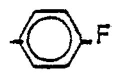 |
| 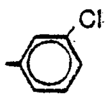 | 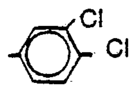 | 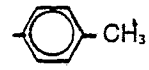 |
| 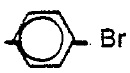 | 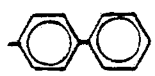 | 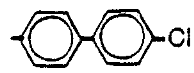 |
| 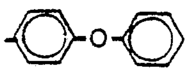 | 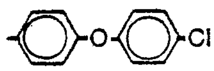 | 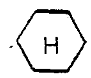 |

Die ausserdem für das erfindungsgemässe Verfahren (a) als Ausgangsstoffe zu verwendenden Aldehyde sind durch die Formel (III) allgemein definiert. In dieser Formel stehen $\underline{R}^2$ und $\underline{R}^3$ für diejenigen Reste, die bereits im Zusammenhang mit der Beschreibung der erfindungsgemässen Stoffe der Formel (I) für diese Substituenten genannt wurden.

Die Aldehyde der Formel (III) sind allgemein bekannte Verbindungen der organischen Chemie. Als Beispele seien die folgenden Verbindungen genannt:

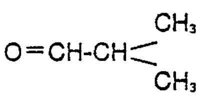 , 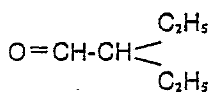 , 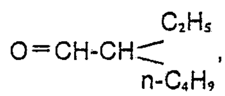 ,

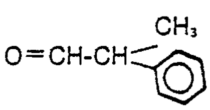 , 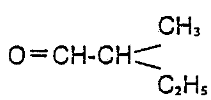 , 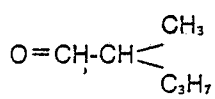 ,

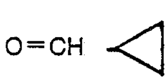 , 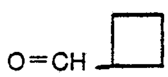 , 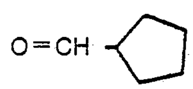 ,

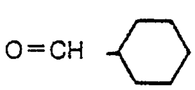 , 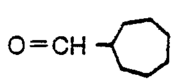 , 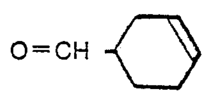 ,

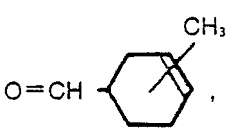 , 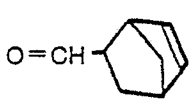 , 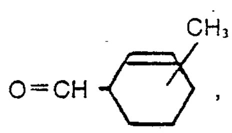 ,

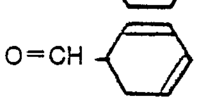 , 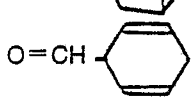 , 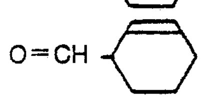 .

Die für das Verfahren (b), Varianten $\alpha$ und $\beta$, als Ausgangsstoffe zu verwendenden Verbindungen sind durch die Formel (Ia) allgemein definiert. In dieser Formel stehen $R^1$, $R^2$ und $R^3$ für diejenigen Reste, die bereits im Zusammenhang mit der Beschreibung der erfindungsgemässen Stoffe der Formel (I) für diese Substituenten genannt wurden.

Verbindungen der Formel (Ia), in denen $R^1$ für gegebenenfalls substituiertes Alkyl oder für Cycloalkyl steht sind erfindungsgemäss, während solche in denen $R^1$ für gegebenenfalls substituiertes Aryl steht, teilweise bekannt sind (vergleiche DE-OS 26 45 617).

Die für das Verfahren (b), Variante $\alpha$, als Reaktionskomponenten benötigten komplexen Hydride sind allgemein bekannte Verbindungen der organischen Chemie. Als Beispiele seien vorzugsweise Natriumborhydrid und Lithiumalanat genannt.

Die für das Verfahren (b), Variante $\beta$, weiterhin als Ausgangsstoffe zu verwendenden Grignard-Verbindungen sind durch die Formel (IV) allgemein definiert.

Die Grignard-Verbindungen der Formel (IV) sind allgemein bekannte Verbindungen der organischen Chemie. Als Beispiele seien genannt: Methylmagnesiumbromid, Ethylmagnesiumbromid, Isopropylmagnesiumbromid und Benzylmagnesiumbromid.

Die für die Verfahren (c), (d) und (e) als Ausgangsstoffe zu verwendenden 1-Vinyltriazol-Derivate sind durch die Formel (Ib) allgemein definiert. In dieser Formel stehen $R^1$, $R^2$, $R^3$ und $R^5$ für diejenigen Reste, die bereits im Zusammenhang mit der Beschreibung der erfindungsgemässen Stoffe der Formel (I) für diese Substituenten genannt wurden.

Die ausserdem für das Verfahren (c) als Ausgangsstoffe zu verwendenden Halogenide sind durch die Formel (V) allgemein definiert.

Die Halogenide der Formel (V) sind allgemein bekannte Verbindungen der organischen Chemie.

Die ausserdem für das Verfahren (d) als Ausgangsstoffe zu verwendenden Säureanhydride sind durch die Formel (VI) allgemein definiert.

Die Säureanhydride der Formel (VI) sind allgemein bekannte Verbindungen der organischen Chemie.

Die ausserdem für das Verfahren (e) als Ausgangsstoffe zu verwendenden Isocyanate sind durch die Formel (VII) allgemein definiert.

Die Isocyanate der Formel (VII) sind allgemein bekannte Verbindungen der organischen Chemie.

Als Lösungsmittel kommen für das erfindungsgemässe Verfahren (a) vorzugsweise inerte organische Lösungsmittel infrage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Das erfindungsgemässe Verfahren (a) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z. B. Bortrifluorid, Bortrichlorid, Zinntetrachlorid oder Titantetrachlorid; organische Basen wie Pyridin und Piperidin; sowie insbesondere Piperidinacetat.

Die Reaktionstemperaturen können bei der Durchführung des erfindungsgemässen Verfahrens (a) in einem grösseren Bereich variiert werden. Im allgemeinen arbeitet man zwischen 20 und 160°C, vorzugsweise bei der Siedetemperatur des jeweiligen Lösungsmittels.

Bei der Durchführung des erfindungsgemässen Verfahrens (a) setzt man auf 1 Mol Triazol-keton der Formel (II) 1 bis 1,5 Mol Aldehyd der Formel (III) und katalytische bis 0,2 molare Mengen an Katalysator ein. Zur Isolierung der Verbindungen der Formel (I) werden die beiden, hinsichtlich der Lage der Doppelbindung isomeren, Reaktionsprodukte nach üblichen Methoden, wie z. B. durch Salzbildung (vergleiche die Herstellungsbeispiele) oder chromatographisch, getrennt. Eine eindeutige Strukturzuordnung erfolgt aufgrund spektroskopischer Daten, insbesondere der NMR-Daten.

Als Lösungsmittel kommen für das erfindungsgemässe Verfahren (b), Variante $\alpha$, vorzugsweise polare organische Lösungsmittel in Frage. Hierzu gehören vorzugsweise Alkohole, wie Methanol, Ethanol, Isopropanol oder Butanol; und Ether, wie Diethylether oder Tetrahydrofuran.

Die Reaktionstemperaturen können bei der Durchführung des erfindungsgemässen Verfahrens (b), Variante $\alpha$, in einem grösseren Bereich variiert werden. Im allgemeinen arbeitet man zwischen 0 bis 30°C, vorzugsweise bei 0 bis 20°C.

Bei der Durchführung des erfindungsgemässen Verfahrens (b), Variante $\alpha$, arbeitet man vorzugsweise mit äquivalenten Mengen an Ausgangsstoffen. Zur Isolierung der Verbindung der Formel (I) wird das Reaktionsgemisch in verdünnter Salzsäure aufgenommen und mit einem organischen Lösungsmittel extrahiert. Die weitere Aufarbeitung erfolgt in üblicher Weise.

Als Lösungsmittel kommen für das erfindungsgemässe Verfahren (b), Variante $\beta$, vorzugsweise wasserfreie Ether, wie Diethylether, Dibtuylether und Tetrahydrofuran infrage.

Die Reaktionstemperaturen können bei der Durchführung des erfindungsgemässen Verfahrens (b), Variante $\beta$, in einem grösseren Bereich variiert werden. Im allgemeinen arbeitet man zwischen 0 und 80°C, vorzugsweise zwischen 30 und 60°C.

Bei der Durchführung des erfindungsgemässen Verfahrens (b), Variante $\beta$, arbeitet man vor-

zugsweise mit äquivalenten Mengen an Ausgangsstoffen. Die Isolierung der Verbindungen der Formel (I) erfolgt in üblicher und allgemein bekannter Weise.

Als Lösungsmittel kommen für das erfindungsgemässe Verfahren (c) vorzugsweise inerte organische Lösungsmittel infrage. Hierzu gehören vorzugsweise Ether, wie Diethylether und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Ketone, wie Aceton oder Methylethylketon; und Nitrile, wie Acetonitril. Der Einfachheit halber kann gegebenenfalls auch ein eingesetztes Säurehalogenid als Lösungsmittel verwendet werden, womit ein entsprechender Überschuss erforderlich wird.

Die Reaktionstemperaturen können bei der Durchführung des erfindungsgemässen Verfahrens (c) in einem grösseren Bereich variiert werden. Im allgemeinen arbeitet man zwischen 20 und 150°C, vorzugsweise zwischen 20 und 100°C, bzw. bei der Siedetemperatur des jeweiligen Lösungsmittels.

Das erfindungsgemässe Verfahren (c) kann gegebenenfalls in Gegenwart einer starken Base durchgeführt werden. Hierzu gehören vorzugsweise Alkalimetall-hydride, Alkalimetallamide und Alkalimetall-alkoholate, wie z. B. Natriumhydrid, Natriumamid und Kalium-tert.-butylat.

Das erfindungsgemässe Verfahren (c) kann gegebenenfalls in Gegenwart von Säurebindern (Halogenwasserstoff-Akzeptoren) durchgeführt werden. Hierzu gehören organische Basen, vorzugsweise tertiäre Amine, wie z. B. Triethylamin; ferner anorganische Basen, wie z. B. Alkalihydroxide und Alkalicarbonate.

Bei der Durchführung des erfingundsgemässen Verfahrens (c) setzt man vorzugsweise auf 1 Mol der Verbindungen der Formel (Ib) 1 bis 3 Mol Halogenid der Formel (V) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

In einer bevorzugten Ausführungsform wird zweckmässigerweise so verfahren, dass man von einer Verbindung der Formel (Ib) ausgeht, letztere in einem geeigneten inerten organischen Lösungsmittel mittels Alkalimetall-hydrid oder Alkalimetall-amid in das Alkenolat überführt, und letzteres ohne Isolierung sofort mit einem Halogenid der Formel (V) umsetzt, wobei unter Austritt von Alkalihalogenid die erfindungsgemässen Verbindungen der Formel (I) in einem Arbeitsgang erhalten werden.

Nach einer weiteren bevorzugten Ausführungsform wird die o. g. bevorzugte Ausführung bei der Umsetzung von Halogeniden der Formel (V), in welchen $R^7$ für Alkyl oder gegebenenfalls substituiertes Aralkyl steht, in einem Zweiphasensystem vorgenommen, wie beispielsweise wässrige Natron- oder Kalilauge/Toluol oder Methylenchlorid, unter Zusatz von 0,01-1 Mol eines Phasen-Transfer-Katalysators, wie beispielsweise einer Ammonium- oder Phosphoniumverbindung, beispielsweise seien Benzyl-dodecyl-dimethyl-ammoniumchlorid (Zephirol) und Triethyl-benzyl-ammoniumchlorid genannt.

Als Lösungsmittel kommen für das erfindungsgemässe Verfahren (d) vorzugsweise inerte organische Lösungsmittel infrage. Hierzu gehören vorzugsweise die beim Verfahren (c) aufgezählten Solventien sowie die jeweils verwendeten Säureanhydride der Formel (VI).

Als Katalysatoren können beim Verfahren (d) vorzugsweise alle üblichen sauren und basischen Katalysatoren verwendet werden, wie z. B. Schwefelsäure, Chlorwasserstoff, Bromwasserstoff, Bortrifuorid, Zinkchlorid, Natriumacetat, Natriumbenzoat, Natriumcarbonat, Calciumoxid, Magnesiumoxid, Pyridin und Triethylamin.

Die Reaktionstemperaturen können bei der Durchführung des erfindungsgemässen Verfahrens (d) in einem grösseren Bereich variiert werden. Im allgemeinen arbeitet man zwischen 20 und 150°C, vorzugsweise zwischen 50 und 120°C.

Bei der Durchführung des Verfahrens (d) arbeitet man vorzugsweise mit äquivalenten Mengen an Ausgangsstoffen. Der Einfachheit halber kann man das eingesetzte Säureanhydrid der Formel (VI) auch als Lösungsmittel verwenden, womit ein entsprechender Überschuss erforderlich wird. Die Isolierung der Verbindungen der Formel (I) erfolgt in üblicher Weise.

Als Lösungsmittel kommen für das erfindungsgemässe Verfahren (e) vorzugsweise inerte organische Lösungsmittel infrage. Hierzu gehören vorzugsweise die beim Verfahren (c) aufgezählten Solventien.

Als Katalysatoren können beim Verfahren (e) vorzugsweise verwendet werden: tertiäre Basen, wie Triethylamin und Pyridin-, oder Zinn-organische Verbindungen, wie Dibutylzinn-dilaurat und Tributylzinn-laurat.

Die Reaktionstemperaturen können bei der Durchführung des erfindungsgemässen Verfahrens (e) in einem grösseren Bereich variiert werden. Im allgemeinen arbeitet man zwischen 0 und 100°C, vorzugsweise zwischen 20 und 40°C.

Bei der Durchführung des erfindungsgemässen Verfahrens (e) arbeitet man vorzugsweise mit äquivalenten Mengen an Ausgangsstoffen. Zur Isolierung der Verbindungen der Formel (I) wird das Lösungsmittel abdestilliert und der Rückstand nach üblichen Methoden aufgearbeitet.

Die nach den Verfahren (a) bis (e) herstellbaren erfindungsgemässen Stoffe der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren in Frage: Die Halogenwasserstoffsäuren, z. B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure, Milchsäure, sowie Sulfonsäure, wie z. B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metall-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisieren reinigen.

Die erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Annwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Ilnteresse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Eine Hemmung des vegetativen Wachstums der Pflanzen kann auch dadurch zu Ertragsteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann auch gleichzeitig zu einer Förderung des generativen Wachstums führen, dadurch dass mehr Assimilate gebildet werden, so dass mehr oder grössere Früchte entstehen.

Ertragssteigerungen können in machen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper

Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, dass z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trokkenheit oder hohen Salzgehalt des Bodens induziert werden. Hierdurch wird die Kultivierung von Pflanzen in Gebieten möglich, die hierzu normalerweise ungeeignet sind.

Besonders gut sind die erfindungsgemässen Verbindungen zur Wuchshemmung von Reis geeignet.

Die erfindungsgemässen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäss verwendbaren Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z. B. gegen den Erreger des Gersten- bzw. des Getreidemehltaus (Erysiphe graminis).

Besonders hervorzuheben ist, dass die erfindungsgemässen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder

Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure. Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweisshydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen, Bodenstrukturverbesserungsmitteln und Pflanzenwachtstumsregulatoren.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemässen Verbindungen als Pflanzenwachstumsregulatoren können die Aufwandmengen in einem grösseren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemässen Stoffe als Pflanzenwachstumsregulatoren gilt, dass die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemässen Stoffe als Fungizide kann die Aufwandmenge je nach Art der Applikation in einem grösseren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

Die gute Wirksamkeit der erfindungsgemässen Stoffe geht aus den nachfolgenden Beispielen hervor.

In diesen Beispielen werden die nachstehend angegebenen Verbindungen als Vergleichssubstanzen eingesetzt:

O
||
A = $Cl-CH_2-CH_2-P-OH$
|
OH

2-Chlorethyl-trimethylammoniumchlorid

B = $Cl-CH_2-CH_2-\overset{\oplus}{N}(CH_3)_3\ Cl^{\ominus}$

2-Chlohethyl-trimethylammoniumchlorid

C = 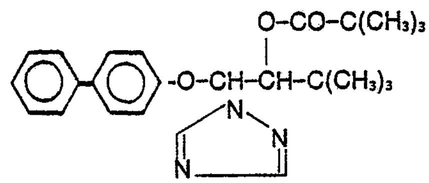

D = 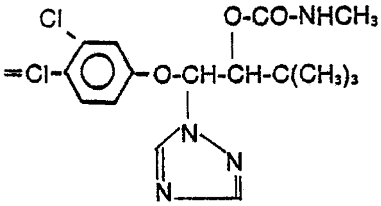

E = 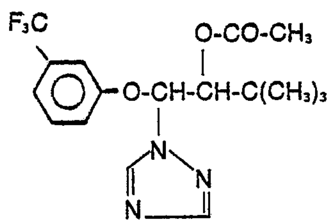

F = 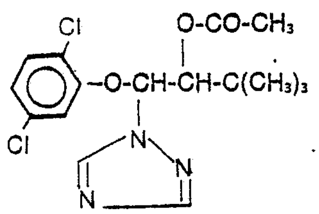

G = 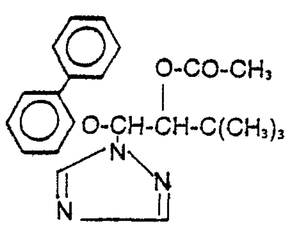

H = 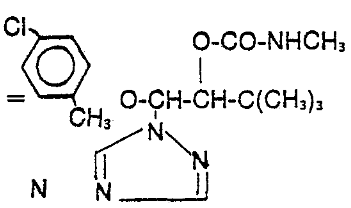

I = 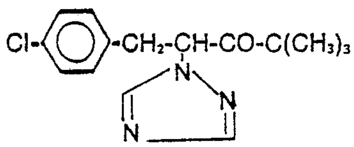

Beispiel A
Ethylenbildung

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Grösse gestanzt. Diese werden zusammen mit 1 ml Wirkstoffzubereitung bzw. Kontrollösung in luftdicht verschliessbare Gefässe gegeben. Nach 24 Stunden wird das Ethylen, das sich in den Gefässen angesammelt hat, mit üblichen Nachweismethoden bestimmt. Die Ethylenentwicklung der mit den Wirkstoffzubereitungen behandelten Blattstücke wird mit derjenigen der Kontrollen verglichen.

Es bedeuten:

0 Ethylenentwicklung wie bei der Kontrolle
\+ leicht erhöhte Ethylenentwicklung
++ stark erhöhte Ethylenentwicklung
+++ sehr stark erhöhte Ethylenentwicklung

Der erfindungsgemässe Wirkstoff (3) zeigt gegenüber der Kontrolle eine stark erhöhte Ethylenentwicklung.

Beispiel B
Wuchshemmung bei Gerste

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 2, 3 und 12 zeigen in diesem Test eine bessere Wuchshemmung als die aus dem Stand der Technik bekannte Substanz (A).

Beispiel C
Wuchsbeeinflussung bei Zuckerrüben

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnass mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemässen Wirkstoffe 2, 3, 4, 12, 14 und 16 zeigen in diesem Test eine bessere Wuchsbeeinflussung als die aus dem Stand der Technik bekannte Substanz (B).

Beispiel D

Wuchshemmung bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 2, 3, 12, 13, 14 und 16 zeigen in diesem Test eine bessere Wachstumshemmung als die aus dem Stand der Technik bekannte Substanz (B).

Beispiel E

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 2, 3, 4 und 12 zeigen in diesem Test gegenüber der Kontrolle eine starke Wuchshemmung.

Beispiel F

Sprossbehandlungs-Test / Getreidemehltau / protektiv
(blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykol-ether) auf und gibt 975 Gewichtsteile Wasser hinzu. Das Konzentrat verdünnt man mit Wasser auf die gewünschte Endkonzentraton der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21-22°C und einer Luftfeuchtigkeit von 80-90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Bei diesem Test zeigen z. B. die folgenden Verbindungen eine sehr gute Wirkung, die derjenigen der aus dem Stand der Technik bekannten Verbindungen (C), (D) und (E) überlegen ist:

Verbindungen gemäss Herstellungsbeispielen: 2, 3, 16, 4, 11 und 12.

Beispiel G

Gerstenmehltau-Test (Erysiphe graminis var. hordei) / systemisch
(pilzliche Getreidesprosskrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulvrigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22°C und 80-90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100%

den gleichen Befallsgrad wie bei der unbehandelten Kontrolle. Der Wirkstoff ist um so wirksamer je geringer der Mehltaubefall ist.

Bei diesem Test zeigen z. B. folgende Verbindungen eine sehr gute Wirkung, die derjenigen der aus dem Stand der Technik bekannten Verbindungen (F), (G) und (H) überlegen ist:

Verbindungen gemäss Herstellungsbeispielen: 2, 3, 4, 11 und 12.

Beispiel H

Myzelwachstums-Test

Verwendeter Nährboden:

| | |
|---|---|
| 20 | Gewichtsteile Agar-Agar |
| 200 | Gewichtsteile Kartoffeldekokt |
| 5 | Gewichtsteile Malz |
| 15 | Gewichtsteile Dextrose |
| 5 | Gewichtsteile Pepton |
| 2 | Gewichtsteile $Na_2HPO_4$ |
| 0,3 | Gewichtsteile $Ca(NO_3)_2$ |

Verhältnis von Lösungsmittelgemisch zum Nährboden:

| | |
|---|---|
| 2 | Gewichtsteile Lösungsmittelgemisch |
| 100 | Gewichtsteile Agarnährboden |

Zusammensetzung Lösungsmittelgemisch:

| | |
|---|---|
| 0,19 | Gewichtsteile DMF oder Aceton |
| 0,01 | Gewichtsteile Emulgator Alkylarylpolyglykolether |
| 1,80 | Gewichtsteile Wasser |
| 2 | Gewichtsteile Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präpartbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach Wachstumsgeschwindigkeit der Pilze nach 4-10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z. B. die folgenden Verbindungen eine sehr gute Wirkung, die derjenigen der aus dem Stand der Technik bekannten Verbindung (I) überlegen ist:

Verbindungen gemäss Herstellungsbeispielen 2 und 3.

Beispiel I

Wuchshemmung bei Reis

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Reispflanzen werden im Gewächshaus in 10×10×10 cm Töpfen in Erde bis zum 2-Blattstadium angezogen. In diesem Stadium werden die Pflanzen tropfnass mit den Wirkstoffzubereitungen besprüht. Nach 10 Tagen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigt der erfindungsgemässe Wirkstoff (2) eine sehr starke wuchshemmende Wirksamkeit.

Beispiel J

Wuchshemmung bei Wasser-Reis var. Nihonbare

5 Teile Wirkstoff werden mit 2,5 Teilen Newkalgen CP-50*), 30 Teilen Bentonit und 62,5 Teilen Talkum in einem Mixer vermischt. 20 Teile Wasser werden zugesetzt. Der Brei wird durch Löcher von 0,5 mm Durchmesser gepresst und getrocknet. Hieraus resultiert ein Granulat mit einer Korngrösse von 0,5 mm Ø und etwa 0,7 mm Länge.

10 Tage alte Pflanzen werden in 25×20×10 cm grosse Schalen gepflanzt, in denen Wasser über der Erde angestaut ist. Nach 10 Tagen wird die Wirkstoffzubereitung ins Wasser appliziert. Nach weiteren 14 Tagen wird die Wuchshöhe der Pflanzen gemessen.

+) 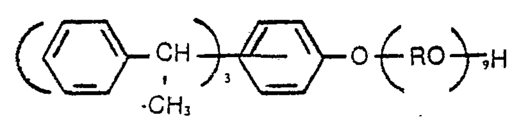

R = Ethylen oder Propylen, im Verhältnis 9 : 1

In diesem Test zeigt der erfindungsgemässe Wirkstoff (2) eine sehr starke wuchshemmende Wirksamkeit.

Herstellungsbeispiele

Beispiel 1

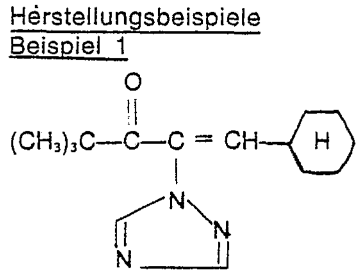

Verfahren (a)

83,5 g (0,5 mol) Pinakolyl-1,2,4-triazol, 60 g (0,54 mol) Cyclohexanaldehyd, 4,2 g (0,05 mol) Piperidin und 6 g (0,1 mol) Eisessig in 300 ml Toluol werden am Wasserabscheider unter Rückfluss erhitzt, bis kein Wasser mehr übergeht. Nach dem Abkühlen der Reaktionslösung wird mit gesättigter Natriumchlorid-Lösung gewaschen, die organische Phase über Natriumsulfat getrocknet, filtriert und eingeengt. Der Rückstand wird in 500 ml Aceton aufgenommen und unter Rühren mit einer filtrierten Lösung von 90 g (0,25 mol) Naphthalin-1,5-disulfonsäure in 500 ml Aceton versetzt.

Der zunächst ausfallende Niederschlag wird abgesaugt, das Filtrat weiter eingeengt und der erhaltene farblose kristalline Rückstand in 500 ml Methylenchlorid aufgenommen. Danach wird halbkonzentrierte Natriumcarbonat-Lösung bis zur alkalischen Reaktion zugegeben. Die organische Phase wird abgetrennt, getrocknet, filtriert und eingeengt. Der ölige Rückstand wird in Petrolether aufgenommen, und der Kristallisation überlassen. Man erhält 64 g (49% der Theorie) 1-Cyclohexyl-4,4-dimethyl-2-(1,2,4-triazol-1-yl)-pent-1-en-3-on vom Schmelzpunkt 98°C.

Herstellung des Ausgangsproduktes

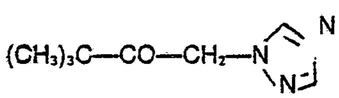

138 g (2 mol) 1,2,4-Triazol werden bei Raumtemperatur portionsweise zu 276,4 g (2 mol) gemahlenem Kaliumcarbonat und 269,2 g (2 mol) α-Chlorpinakolin in 500 ml Aceton gegeben, wobei die Innentemperatur bis zur Siedehitze ansteigt. Man lässt 5 Stunden unter Rückfluss rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Thorie) 3,3-Dimethyl-1-(1,2,4-triazol-1-yl)-butan-2-on vom Schmelzpunkt 62-64°C.

Beispiel 2

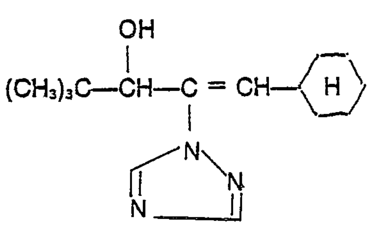

Verfahren (b), Variante α:

26 g (0,1 mol) 1-Cyclohexyl-4,4-dimethyl-2-(1,-2,4-triazol-1-yl)-pent-1-en-3-on (Beispiel 1) werden in 200 ml Methanol aufgenommen und unter Rühren und Kühlen portionsweise mit 4,5 g Natriumborhydrid versetzt. Nach beendeter Reaktion wird das Reaktionsgemisch auf pH-6 eingestellt und eingeengt. Der Rückstand wird in 200 ml Methylenchlorid aufgenommen, mit gesättigter Natriumhydrogencarbonat-Lösung gewaschen, über Natriumsulfat getrocknet, filtriert und eingeengt. Der Rückstand wird aus Petrolether umkristallisiert. Man erhält 14,5 g (55% der Thorie) 1-Cyclohexyl-4,4-dimethyl-2-(1,-2,4-triazol-1-yl)-pent-1-en-3-ol vom Schmelzpunkt 131°C.

Beispiel 3

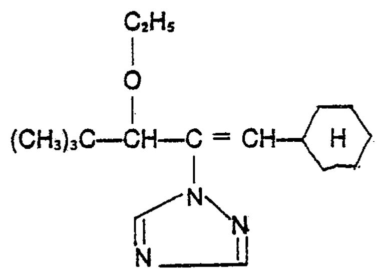

(Verfahren c)

Eine Lösung von 26,3 g (0,1 mol) 1-Cyclohexyl--4,4-dimethyl-2-(1,2,4-triazol-1-yl)-pent-1-en-3-ol (Beispiel 2) in 50 ml Dioxan wird zu einer Suspension von 3 g 80%igem Natriumhydrid in 100 ml Dioxan getropft. Das Gemisch wird nach beendeter Zugabe 1 Stunde auf 50°C erwärmt. Nach dem Abkühlen werden 10,9 g (0,1 mol) Ethylbromid zugetropft und das Reaktionsgemisch über Nacht unter Rückfluss erhitzt. Nach dem Abkühlen werden 10 ml Methanol zugegeben und das Gemisch am Rotationsverdampfer eingeengt. Der Rückstand wird in Methylenchlorid aufgenommen und mit Wasser gewaschen. Nach dem Trocknen der organischen Phase über Natriumsulfat wird filtriert und das Filtrat eingeengt. Der Rückstand wird destilliert. Man erhält 11,0 g (37,8% der Theorie) 1-Cyclohexyl-3--ethoxy-4,4-dimethyl-2-(1,2,4-triazol-1-yl)-pent-1--en vom Siedepunkt 110°C/0,07 mm.

Beispiel 4

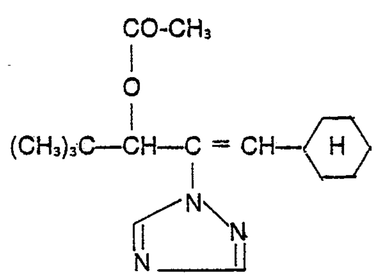

Verfahren c)

Eine Lösung von 13,15 g (0,05 mol) 1-Cyclohexyl-4,4-dimethyl-2-(1,2,4-triazol-1-yl)-pent-1-en--3-ol (Beispiel 2) in 50 ml Dioxan wird zu einer Suspension von 1,5 g 80%igem Natriumhydrid in 50 ml Dioxan getropft. Nach beendeter Wasserstoffentwicklung werden 3,9 g (0,05 mol) Acetylchlorid zugetropft. Das Gemisch wird 4 Stunden unter Rückfluss erhitzt. Das Lösungsmittel wird nach dem Abkühlen im Vakuum abdestilliert, der Rückstand in Methylenchlorid aufgenommen und mit Wasser extrahiert. Die organische Phase wird über Natriumsulfat getrocknet, filtriert und die Lösung eingeengt. Der Rückstand wird über eine Säule (Kieselgel; Methanol: Chloroform = 1:3) gereinigt.

Man erhält 5,6 g (35,4% der Theorie) 3-Acetoxy-1-cyclohexyl-4,4-dimethyl-2-(1,2,4-triazol-1-yl)-pent-1-en als schwach gelbes Öl.

(Verfahren d)

Zu einer Lösung von 13,15 g (0,05 mol) 1-Cyclohexyl-4,4-dimethyl-2-(1,2,4-triazol-1-yl)-pent-1-en-3-ol (Beispiel 2) in 100 ml Acetanhydrid werden 2 ml Pyridin gegeben. Das Gemisch wird vier Stunden bei 70°C gerührt. Danach wird das Reaktionsgemisch auf Wasser gegossen und mit Natriumhydrogencarbonat neutralisiert. Die wässrige Phase wird mehrmals mit Ether extrahiert. Die vereinigten Etherphasen werden über Natriumsulfat getrocknet und eingeengt. Man erhält 11,2 g (70,8% der Theorie) 3-Acetoxy-1-cyclohexyl-4,4-dimethyl-2-(1,2,4-triazol-1-yl)-pent-1-en als schwach gelbes Öl.

Beispiel 5

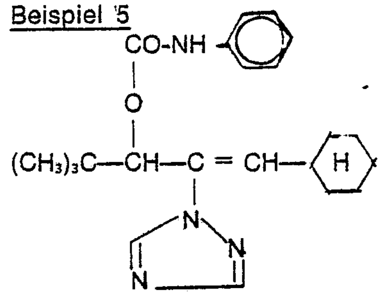

(Verfahren e)

Zu einer Lösung von 13,15 g (0,05 mol) 1-Cyclohexyl-4,4-dimethyl-2-(1,2,4-triazol-1-yl)-pent-1-en-3-ol (Beispiel 2) in 100 ml Ether werden 6,5 g (0,055 mol) Phenylisocyanat und drei Tropfen Tributylzinnlaurat als Katalysator gegeben. Das Gemisch wird 5 Tage bei Raumtemperatur gerührt. Nach Abdestillieren des Lösungsmittels im Vakuum wird der Rückstand aus Essigester/Ligroin umkristallisiert. Man erhält 4,8 g (25,1% der Theorie) 1-Cyclohexyl-4,4-dimethyl-3-phenylcarbamoyloxy-2-(1,2,4-triazol-1-yl)-pent-1-en vom Schmelzpunkt 156°C.

In analoger Weise werden die nachfolgenden Verbindungen der Tabelle 1 erhalten.

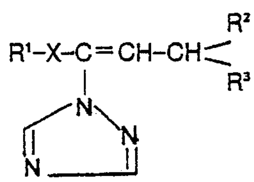

(I)

| Bsp. Nr. | $R^1$ | X | $R^2$ | $R^3$ | Schmelzpunkt (°C) |
|---|---|---|---|---|---|
| 6 | $(CH_3)_3C$ | -CO- | 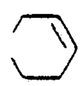 | | 193 ($\frac{1}{2}$ × NDS) |
| 7 | $(CH_3)_3C$ | -CO- | 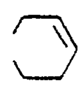 | | 40-48 |
| 8 | $(CH_3)_3C$ | -CO- | 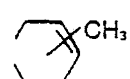 | | 49 |
| 9 | $(CH_3)_3C$ | -CO- | 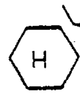 | | 201 ($\frac{1}{2}$ × NDS) |
| 10 | $(CH_3)_3C$ | -CO- | n-$C_4H_9$ | $C_2H_5$ | Öl |
| 11 | $(CH_3)_3C$ | -CH(OH)- | 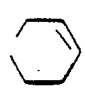 | | 151 (Z-Form) |
| 12 | $(CH_3)_3C$ | -CH(OH)- | 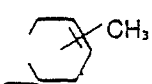 | | Öl |
| 13 | $(CH_3)_3C$ | -CH($OC_3H_7$-n)- | 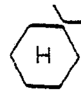 | | Öl |

| Bsp. Nr. | $R^1$ | X | $R^2$ | $R^3$ | Schmelz-punkt (°C) |
|---|---|---|---|---|---|
| 14 | $(CH_3)_3C$ | -CH(O-CO- 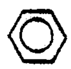 )- | 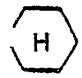 | | Öl |
| 15 | $(CH_3)_3C$ | $-CH(O-CO-CHCl_2)-$ | 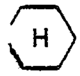 | | Öl |
| 16 | $(CH_3)_3C$ | -CH(O-CO- 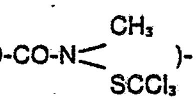 | 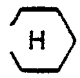 | | Öl |
| 17 | 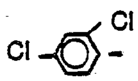 | -CH(OH)- | 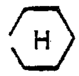 | | Öl |
| 18 | 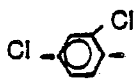 | -CH(OH)- | $C_2H_5$ | $C_2H_5$ | Öl |
| 19 | 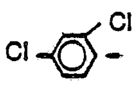 | -CH(OH)- | 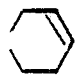 | | Öl |
| 20 | 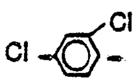 | $-CH(O-COCH_3)-$ | 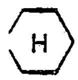 | | Öl |
| 21 | $(CH_3)_3C$ | $-CCH_3(OH)-$ | 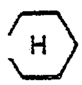 | | 101 |
| 22 | $(CH_3)_3C$ | -CH(OH)- | 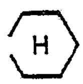 | | 154 (. HCl) (Z-Form) |
| 23 | 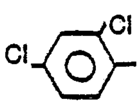 | -CH(OH)- | $C_3H_7$ | $CH_3$ | Öl |
| 24 | $(CH_3)_3C$ | -CH(OH)- | 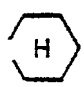 | | 110 (. $CuCl_2$) (Z-Form) |
| 25 | 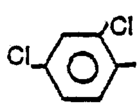 | $-CH(O-CO-NHCH_3)-$ | 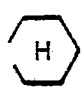 | | 62 |
| 26 | 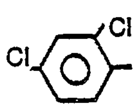 | -CH(OH)- | $C_2H_5$ | $CH_3$ | Öl |
| 27 | 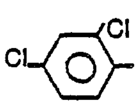 | -CH(O-CO- 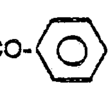 )- | $C_3H_7$ | $CH_3$ | Öl |
| 28 | 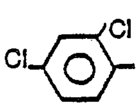 | $-CH(O-CO-CH_3)-$ | $C_3H_7$ | $CH_3$ | Öl |

| Bsp. Nr. | $R^1$ | X | $R^2$ | $R^3$ | Schmelzpunkt (°C) |
|---|---|---|---|---|---|
| 29 | $(CH_3)_3C$ | -CH(O-CH$_2$- 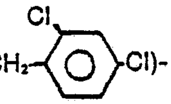 )- | 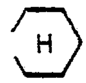 | | Öl (Z-Form) |
| 30 | $ClCH_2$-$C(CH_3)_2$- | -CO- | 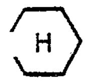 | | 51 |
| 31 | $ClCH_2$-$C(CH_3)_2$- | -CO- | 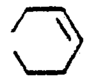 | | Öl |
| 32 | $ClCH_2$-$C(CH_3)_2$- | -CH(OH)- | 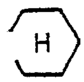 | | Öl |
| 33 | 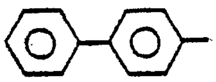 | -CH(OH)- | 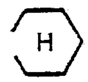 | | 156 |
| 34 | $(CH_3)_3C$ | -CH(OH)- | 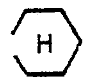 | | 153 (. $HNO_3$) (Z-Form) |
| 35 | 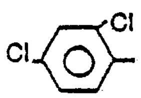 | -CH(OH)- | 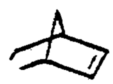 | | Öl |
| 36 | 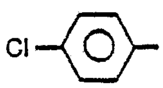 | -CH(OH)- | 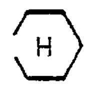 | | Öl |
| 37 | 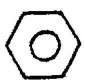 | -CH(OH)- | 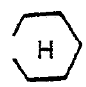 | | Öl |
| 38 | 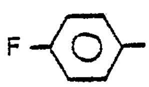 | -CH(OH)- | $C_2H_5$ | $CH_3$ | Öl |
| 39 | 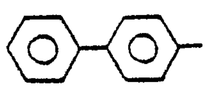 | -CH(OH)- | $C_2H_5$ | $CH_3$ | Öl |
| 40 | 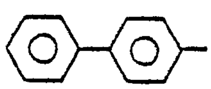 | -CH(OH)- | $C_4H_9$ | $C_2H_5$ | Öl |
| 41 | $ClCH_2$-$C(CH_3)_2$- | -CH(OH)- | 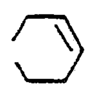 | | Öl |
| 42 | $(CH_3)_3C$ | -CH($OCH_3$)- | 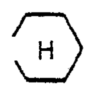 | | 63 (Z-Form) |
| 43 | 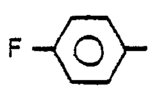 | -CH(OH)- | $C_4H_9$ | $C_2H_5$ | Öl |
| 44 | $FCH_2$-$C(CH_3)_2$- | -CO- | $C_4H_9$ | $C_2H_5$ | Öl |
| 45 | $(CH_3)_3C$ | -CH($OCH_3$)- | 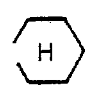 | | 104 (E-Form) |

| Bsp. Nr. | $R^1$ | X | $R^2$ | $R^3$ | Schmelz-punkt (°C) |
|---|---|---|---|---|---|
| 46 | $(CH_3)_3C$ | -CH(OH)- | 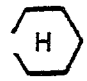 | | 137 (. $HNO_3$) (E-Form) |
| 47 | 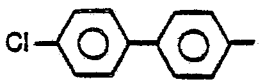 | -CH(OH)- | $CH_3$ | $CH_3$ | 187 |
| 48 | $ClCH_2$-$C(CH_3)_2$- | -CH(OH)- | $CH_3$ | $CH_3$ | Öl |
| 49 | $(CH_3)_3C$ | -CH(OH)- | 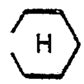 | | 242 (. $\frac{1}{2}$ NDS) (E-Form) |
| 50 | $(CH_3)_3C$ | -CH(OH)- | 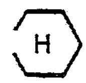 | | 168 (. $CuCl_2$) (E-Form) |
| 51 | $(CH_3)_3C$ | -CO- | 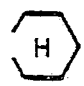 | | 137-140 (. $CuCl_2$) (E-Form) |
| 52 | 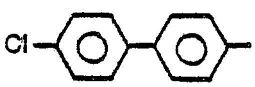 | -CH(OH)- | 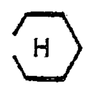 | | 157 |
| 53 | 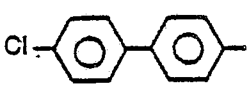 | -CH(OH)- | $C_4H_9$ | $C_2H_5$ | 118 |
| 54 | $FCH_2$-$C(CH_3)_2$- | -CO- | 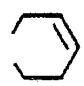 | | Öl |
| 55 | $FCH_2$-$C(CH_3)_2$- | -CO- | 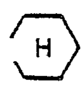 | | Öl |
| 56 | $FCH_2$-$C(CH_3)_2$- | -CH(OH)- | 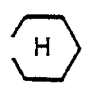 | | Öl |
| 57 | $FCH_2$-$C(CH_3)_2$- | -CH(OH)- | 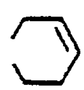 | | Öl |
| 58 | $FCH_2$-$C(CH_3)_2$- | -CO- | 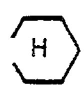 | | Öl (Z-Form) |
| 59 | $FCH_2$-$C(CH_3)_2$- | -CO- | 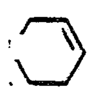 | | Öl (Z-Form) |
| 60 | $ClCH_2$-$C(CH_3)_2$- | -CO- | 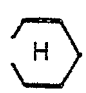 | | 103 (E-Form) |
| 61 | 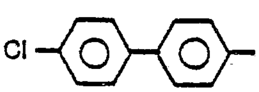 | -CH(OH)- | | | 144 |

| Bsp. Nr. | $R^1$ | X | $R^2$ | $R^3$ | Schmelz-punkt (°C) |
|---|---|---|---|---|---|
| 62 | Cl 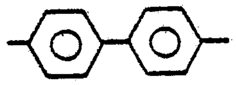 | -CH(OH)- | $C_2H_5$ | $C_2H_5$ | 148 |
| 63 | $FCH_2$-$C(CH_3)_2$- | -CH(OH)- | 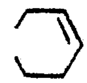 | | $n_D^{20}$: 1,5049 (Z-Form) |
| 64 | $FCH_2$-$C(CH_3)_2$- | -CH(OH)- | 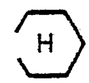 | | $n_D^{20}$: 1,4910 (Z-Form) |
| 65 | $ClCH_2$-$C(CH_3)_2$- | -CH(OH)- | 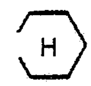 | | $n_D^{20}$: 1,5050 (E-Form) |

E- und Z-Form = Die beiden möglichen geometrischen isomeren Formen
NDS = 1,5-Naphthalindisulfonsäure

**Patentansprüche**

1. 1-Vinyltriazole der Formel

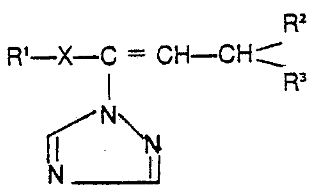
(I)

in welcher

$R^1$ für gegebenenfalls durch Halogen, Alkylcarbonyloxy mit 1 bis 4 Kohlenstoffatomen im Alkylteil, Alkylsulfonyloxy mit 1 bis 4 Kohlenstoffatomen und/oder Phenylsulfonyloxy, welches durch Halogen und/oder Alkyl mit 1 bis 4 Kohlenstoffatomen substituiert sein kann, substituiertes Alkyl mit 1 bis 4 Kohlenstoffatomen, Cycloalkyl mit 5 bis 7 Kohlenstoffatomen sowie Aryl mit 6 bis 10 Kohlenstoffatomen, wobei diese Aryl-Reste substituiert sein können durch Halogen, Alkyl mit 1 bis 4 Kohlenstoffatomen, Phenyl, Phenoxy, Halogenphenyl und/oder Halogenphenoxy, steht,

$R^2$ für Alkyl mit 1 bis 4 Kohlenstoffatomen steht,

$R^3$ für Alkyl mit 1 bis 4 Kohlenstoffatomen, Cycloalkyl mit 5 bis 7 Kohlenstoffatomen, gegebenenfalls durch Alkyl mit 1 bis 4 Kohlenstoffatomen substituiertes Cycloalkenyl mit 5 bis 7 Kohlenstoffatomen, Alkenyl mit 2 bis 4 Kohlenstoffatomen oder für gegebenenfalls durch Halogen und/oder Alkyl mit 1 bis 4 Kohlenstoffatomen substituiertes Aryl mit 6 bis 10 Kohlenstoffatomen steht, $R^2$ und $R^3$ ausserdem gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen oder Cycloalkyl mit 3 bis 7 Kohlenstoffatomen stehen,

X für die Gruppe $-C(OR^4)R^5-$ sowie zusätzlich für die Ketogruppe steht, wenn $R^1$ für gegebenenfalls substituiertes Alkyl mit 1 bis 4 Kohlenstoffatomen oder Cycloalkyl steht,

$R^4$ für Wasserstoff, Alkyl mit 1 bis 4 Kohlenstoffatomen, gegebenenfalls durch Halogen, Alkyl mit 1 bis 4 Kohlenstoffatomen, Halogenalkyl mit bis zu 2 Kohlenstoffatomen und bis zu 3 gleichen oder verschiedenen Halogenatomen und/oder Phenyl oder Phenoxy, welche ihrerseits durch Halogen substituiert sein können, substituiertes Aralkyl mit 1 oder 2 Kohlenstoffatomen im Alkylteil und 6 bis 10 Kohlenstoffatomen im Arylteil, für den Acylrest $-CO-R^{10}$ oder den Carbamoylrest $-CO-NR^{11}R^{12}$ steht,

$R^5$ für Wasserstoff, Alkyl mit 1 bis 4 Kohlenstoffatomen sowie für gegebenenfalls durch Halogen oder Alkyl mit 1 bis 4 Kohlenstoffatomen substituiertes Aralkyl mit 1 oder 2 Kohlenstoffatomen im Alkylteil und 6 bis 10 Kohlenstoffatomen im Arylteil steht,

$R^{10}$ für Alkyl mit 1 bis 4 Kohlenstoffatomen, Halogenalkyl mit 1 oder 2 Kohlenstoffatomen und 1 bis 5 gleichen oder verschiedenen Halogenatomen sowie für gegebenenfalls durch Halogen und/oder Alkyl mit 1 bis 4 Kohlenstoffatomen substituiertes Phenyl oder Benzyl steht,

$R^{11}$ für Wasserstoff oder Alkyl mit 1 bis 4 Kohlenstoffatomen steht und

$R^{12}$ für Alkyl mit 1 bis 8 Kohlenstoffatomen, Halogenalkyl mit bis zu 4 Kohlenstoffatomen und bis zu 5 gleichen oder verschiedenen Halogenatomen, Halogenalkylmercapto mit bis zu 2 Kohlenstoffatomen und bis zu 5 Halogenatomen sowie für gegebenenfalls durch Halogen, Alkyl mit 1 bis 4 Kohlenstoffatomen und/oder Halogenalkyl mit bis zu 2 Kohlenstoffatomen und bis zu 5 gleichen oder verschiedenen Halogenatomen substituiertes Aryl mit 6 bis 10 Kohlenstoffatomen steht,

sowie deren Säureadditions-Salze und Metallsalz-Komplexe.

2. 1-Cyclohexyl-4,4-dimethyl-2-(1,2,4-triazol-1-yl)-pent-1-en-3-on der Formel

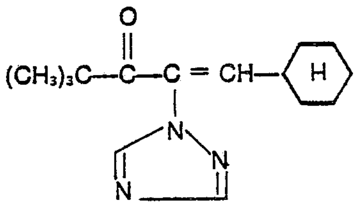

3. 1-Cyclohexyl-4,4-dimethyl-2-(1,2,4-triazol-1-yl)-pent-1-en-3-ol der Formel

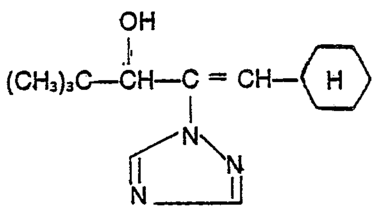

4. 1-Cyclohexyl-3-ethoxy-4,4-dimethyl-2-(1,2,4-triazol-1-yl)-pent-1-en der Formel

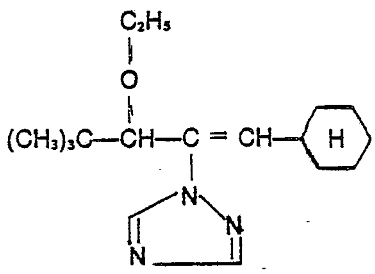

5. Verfahren zur Herstellung von 1-Vinyl-triazolen der Formel

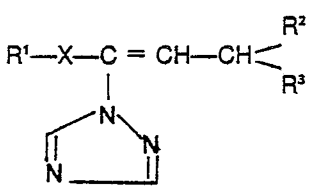 (I)

in welcher

$R^1$ für gegebenenfalls durch Halogen, Alkylcarbonyloxy mit 1 bis 4 Kohlenstoffatomen im Alkylteil, Alkylsulfonyloxy mit 1 bis 4 Kohlenstoffatomen und/oder Phenylsulfonyloxy, welches durch Halogen und/oder Alkyl mit 1 bis 4 Kohlenstoffatomen substituiert sein kann, substituiertes Alkyl mit 1 bis 4 Kohlenstoffatomen, Cycloalkyl mit 5 bis 7 Kohlenstoffatomen sowie Aryl mit 6 bis 10 Kohlenstoffatomen, wobei diese Aryl-Reste substituiert sein können durch Halogen, Alkyl mit 1 bis 4 Kohlenstoffatomen, Phenyl, Phenoxy, Halogenphenyl und/oder Halogenphenoxy, steht, $R^2$ für Alkyl mit 1 bis 4 Kohlenstoffatomen steht, $R^3$ für Alkyl mit 1 bis 4 Kohlenstoffatomen, Cycloalkyl mit 5 bis 7 Kohlenstoffatomen, gegebenenfalls durch Alkyl mit 1 bis 4 Kohlenstoffatomen substituiertes Cycloalkenyl mit 5 bis 7 Kohlenstoffatomen, Alkenyl mit 2 bis 4 Kohlenstoffatomen oder für gegebenenfalls durch Halogen und/oder Alkyl mit 1 bis 4 Kohlenstoffatomen substituiertes Aryl mit 6 bis 10 Kohlenstoffatomen steht, $R^2$ und $R^3$ ausserdem gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen oder Cycloalkyl mit 3 bis 7 Kohlenstoffatomen stehen, X für die Gruppe $-C(OR^4)R^5-$ sowie zusätzlich für die Ketogruppe steht, wenn $R^1$ für gegebenenfalls substituiertes Alkyl mit 1 bis 4 Kohlenstoffatomen oder Cycloalkyl steht, $R^4$ für Wasserstoff, Alkyl mit 1 bis 4 Kohlenstoffatomen, gegebenenfalls durch Halogen, Alkyl mit 1 bis 4 Kohlenstoffatomen, Halogenalkyl mit bis zu 2 Kohlenstoffatomen und bis zu 3 gleichen oder verschiedenen Halogenatomen und/oder Phenyl oder Phenoxy, welche ihrerseits durch Halogen substituiert sein können, substituiertes Aralkyl mit 1 oder 2 Kohlenstoffatomen im Alkylteil und 6 bis 10 Kohlenstoffatomen im Arylteil, für den Acylrest $-CO-R^{10}$ oder den Carbamoylrest $-CO-NR^{11}R^{12}$ steht, $R^5$ für Wasserstoff, Alkyl mit 1 bis 4 Kohlenstoffatomen sowie für gegebenenfalls durch Halogen oder Alkyl mit 1 bis 4 Kohlenstoffatomen substituiertes Aralkyl mit 1 oder 2 Kohlenstoffatomen im Alkylteil und 6 bis 10 Kohlenstoffatomen im Arylteil steht, $R^{10}$ für Alkyl mit 1 bis 4 Kohlenstoffatomen, Halogenalkyl mit 1 oder 2 Kohlenstoffatomen und 1 bis 5 gleichen oder verschiedenen Halogenatomen sowie für gegebenenfalls durch Halogen und/oder Alkyl mit 1 bis 4 Kohlenstoffatomen substituiertes Phenyl oder Benzyl steht, $R^{11}$ für Wasserstoff oder Alkyl mit 1 bis 4 Kohlenstoffatomen steht und $R^{12}$ für Alkyl mit 1 bis 8 Kohlenstoffatomen, Halogenalkyl mit bis zu 4 Kohlenstoffatomen und bis zu 5 gleichen oder verschiedenen Halogenatomen, Halogenalkylmercapto mit bis zu 2 Kohlenstoffatomen und bis zu 5 Halogenatomen sowie für gegebenenfalls durch Halogen, Alkyl mit 1 bis 4 Kohlenstoffatomen und/oder Halogenalkyl mit bis zu 2 Kohlenstoffatomen und bis zu 5 gleichen oder verschiedenen Halogenatomen substituiertes Aryl mit 6 bis 10 Kohlenstoffatomen steht,

sowie deren Säureadditions-Salzen und Metallsalz-Komplexen, dadurch gekennzeichnet, dass man

a) Triazol-Ketone der Formel

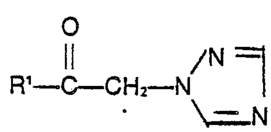 (II)

in welcher

$R^1$ die oben angegebene Bedeutung hat, mit Aldehyden der Formel

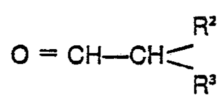 (III)

in welcher

$R^2$ und $R^3$ die oben angegebene Bedeutung haben, in Gegenwart eines Lösungsmittels und in Gegenwart eines Katalysators umsetzt und von den aufgrund der Wasserabspaltung sich bildenden Isomeren das gewünschte isomere Produkt der Formel

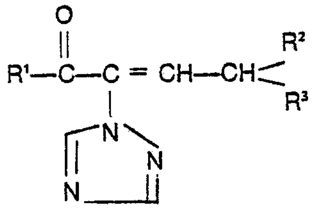

(Ia)

in welcher

$R^1$, $R^2$ und $R^3$ die oben angegebene Bedeutung haben, nach üblichen Methoden isoliert, oder

b) nach dem Verfahren a) erhältliche Verbindungen der Formel

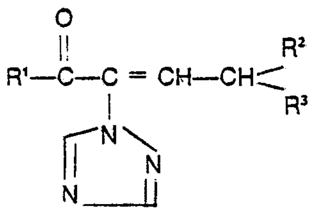

(Ia)

in welcher

$R^1$, $R^2$ und $R^3$ die oben angegebene Bedeutung haben, entweder

α) mit komplexen Hydriden in Gegenwart eines Lösungsmittels reduziert oder

β) mit Grignard-Verbindungen der Formel

$R^6$—Mg—Hal (IV)

in welcher

$R^6$ für Alkyl mit 1 bis 4 Kohlenstoffatomen oder für gegebenenfalls durch Halogen oder Alkyl mit 1 bis 4 Kohlenstoffatomen substituiertes Aralkyl mit 1 bis 2 Kohlenstoffatomen im Alkylteil und 6 bis 10 Kohlenstoffatomen im Arylteil steht und

Hal für Chlor, Brom oder Jod steht, in Gegenwart eines Lösungsmittels reduziert, oder

c) nach dem Verfahren (b), Varianten α und β, erhältliche Verbindungen der Formel

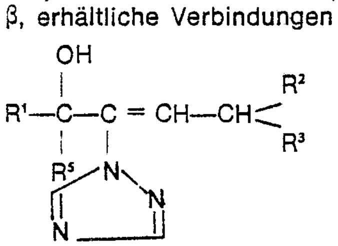

(Ib)

in welcher

$R^1$, $R^2$, $R^3$ und $R^5$ die oben angegebene Bedeutung haben, mit Halogen der Formel

$R^7$—Hal' (V)

in welcher

$R^7$ für Alkyl mit 1 bis 4 Kohlenstoffatomen, gegebenenfalls ein- oder mehrfach, gleich oder verschieden durch Halogen, Alkyl mit 1 bis 4 Kohlenstoffatomen, Halogenalkyl mit bis zu 2 Kohlenstoffatomen und bis zu 3 Halogenatomen, gegebenenfalls durch Halogen substituiertes Phenyl oder durch Phenoxy substituiertes Aralkyl mit 1 bis 2 Kohlenstoffatomen im Alkylteil und 6 bis 10 Kohlenstoffatomen im Arylteil, oder für die Reste der Formeln -CO-$R^{10}$ und -CO-$NR^{11}$-$R^{12}$, in welchen $R^{10}$, $R^{11}$ und $R^{12}$ di oben angegebene Bedeutung haben, und

Hal' für Fluor, Chlor oder Brom steht, in Gegenwart eines Lösungsmittels und gegebenenfalls in Gegenwart einer starken Base bzw. gegebenenfalls in Gegenwart eines Säurebindemittels umsetzt, oder

d) nach dem Verfahren (b), Varianten α und β, erhältliche Verbindungen der Formel

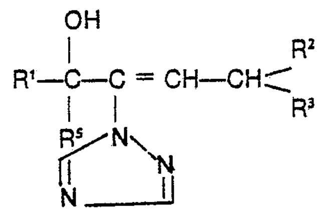

(Ib)

in welcher

$R^1$, $R^2$, $R^3$ und $R^5$ die oben angegebene Bedeutung haben, mit Säureanhydriden der Formel

$R^8$—O—$R^8$ (VI)

in welcher

$R^8$ für den Acylrest der Formel -CO-$R^{10}$ steht, worin

$R^{10}$ die oben angegebene Bedeutung hat, in Gegenwart eines Lösungsmittels und gegebenenfalls in Gegenwart eines Katalysators umsetzt, oder

e) nach dem Verfahren (b), Varianten α und β, erhältliche Verbindungen der Formel

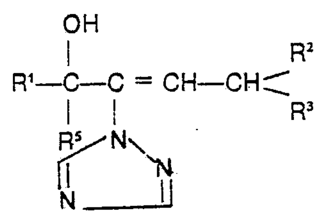

(Ib)

mit Isocyanaten der Formel

O=C=N—$R^9$ (VII)

in welcher

$R^9$ für Alkyl mit 1 bis 8 Kohlenstoffatomen, Halogenalkyl mit bis zu 4 Kohlenstoffatomen und bis zu 5 gleichen oder verschiedenen Halogenatomen oder für gegebenenfalls einfach oder mehrfach gleich oder verschieden durch Halogen, Alkyl mit 1 bis 4 Kohlenstoffatomen, Halogenalkyl mit bis zu 2 Kohlenstoffatomen und bis zu 5 gleichen oder verschiedenen Halogenatomen oder Halogenalkylmercapto mit 1 bis 2 Kohlenstoffatomen und bis zu 5 Halogenatomen substituiertes Aryl mit 6 bis 10 Kohlenstoffatomen steht, in Gegenwart eines Lösungsmittels und gegebenenfalls in Gegenwart eines Katalysators umsetzt, und gegebenenfalls anschliessend an die nach den Verfahren (a) bis (e) erhältlichen 1-Vinyltriazol-Derivate eine Säure oder ein Metall-Salz addiert.

6. Pflanzenwachstumsregulierende und fungizide Mittel, gekennzeichnet durch einen Gehalt an mindestens einem 1-Vinyltriazol-Derivat der Formel (I) gemäss Anspruch 1 bzw. einem Säureadditions-Salz oder Metallsalz-Komplex eines 1-Vinyltriazol-Derivates der Formel (I) gemäss Anspruch 1.

7. Verwendung von 1-Vinyltriazol-Derivaten der Formel (I) gemäss Anspruch 1 bzw. von Säureadditions-Salzen oder Metallsalz-Komplexen von 1-Vinyltriazol-Derivaten der Formel (I) gemäss Anspruch 1 zur Regulierung des Pflanzenwachstums bzw. zur Bekämpfung von Pilzen.

8. Verfahren zur Herstellung von pflanzenwachstumsregulierenden Mitteln bzw. von fungiziden Mitteln, dadurch gekennzeichnet, dass man 1-Vinyltriazol-Derivate der Formel (I) gemäss Anspruch 1 bzw. Säureadditions-Salze oder Metallsalz-Komplexe von 1-Vinyltriazol-Derivaten der Formel (I) gemäss Anspruch 1 mit Streckmitteln und/oder oberflächenaktiven Stoffen vermischt.

**Claims**

1. 1-Vinyltriazoles of the formula

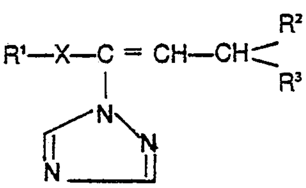

(I)

in which

$R^1$ represents alkyl with 1 to 4 carbon atoms which is optionally substituted by halogen, alkylcarbonyloxy with 1 to 4 carbon atoms in the alkyl part, alkylsulphonyloxy with 1 to 4 carbon atoms and/or phenylsulphonyloxy, which can be substituted by halogen and/or alkyl with 1 to 4 carbon atoms, cycloalkyl with 5 to 7 carbon atoms or aryl with 6 to 10 carbon atoms, it being possible for these aryl radicals to be substituted by halogen, alkyl with 1 to 4 carbon atoms, phenyl, phenoxy, halogenophenyl and/or halogenophenoxy,

$R^2$ represents alkyl with 1 to 4 carbon atoms,

$R^3$ represents alkyl with 1 to 4 carbon atoms, cycloalkyl with 5 to 7 carbon atoms, cycloalkenyl with 5 to 7 carbon atoms which is optionally substituted by alkyl with 1 to 4 carbon atoms, alkenyl with 2 to 4 carbon atoms or aryl with 6 to 10 carbon atoms which is optionally substituted by halogen and/or alkyl with 1 to 4 carbon atoms, $R^2$ and $R^3$, together with the carbon atom to which they are bonded, also represent cycloalkenyl with 5 to 7 carbon atoms which is optionally substituted by alkyl with 1 to 4 carbon atoms or cycloalkyl with 3 to 7 carbon atoms,

X represents the group $-C(OR^4)R^5$ and additionally represents the keto group if $R^1$ represents optionally substituted alkyl with 1 to 4 carbon atoms or cycloalkyl,

$R^4$ represents hydrogen, alkyl with 1 to 4 carbon atoms, aralkyl with 1 or 2 carbon atoms in the alkyl part and 6 to 10 carbon atoms in the aryl part which is optionally substituted by halogen, alkyl with 1 to 4 carbon atoms, halogenalkyl with up to 2 carbon atoms and up to 3 identical or different halogen atoms and/or phenyl or phenoxy which in their turn can be substituted by halogen, the acyl radical $-CO-R^{10}$ or the carbamoyl radical $-CO-NR^{11}R^{12}$,

$R^5$ represents hydrogen, alkyl with 1 to 4 carbon atoms or aralkyl with 1 or 2 carbon atoms in the alkyl part and 6 to 10 carbon atoms in the aryl part which is optionally substituted by halogen or alkyl with 1 to 4 carbon atoms,

$R^{10}$ represents alkyl with 1 to 4 carbon atoms, halogenalkyl with 1 or 2 carbon atoms and 1 to 5 identical or different halogen atoms or phenyl or benzyl which are optionally substituted by halogen and/or alkyl with 1 to 4 carbon atoms,

$R^{11}$ represents hydrogen or alkyl with 1 to 4 carbon atoms and

$R^{12}$ represents alkyl with 1 to 8 carbon atoms, halogenalkyl with up to 4 carbon atoms and up to 5 identical or different halogen atoms, halogenoalkylmercapto with up to 2 carbon atoms and up to 5 halogen atoms or aryl with 6 to 10 carbon atoms which is optionally substituted by halogen, alkyl with 1 to 4 carbon atoms and/or halogenoalkyl with up to 2 carbon atoms and up to 5 identical or different halogen atoms, and acid addition salts and metal salt complexes thereof.

2. 1-Cyclohexyl-4,4-dimethyl-2-(1,2,4-triazol-1-yl)-pent-1-en-3-one of the formula

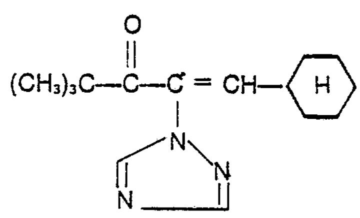

3. 1-Cyclohexyl-4,4-dimethyl-2-(1,2,4-triazol-1-yl)-pent-1-en-3-ol of the formula

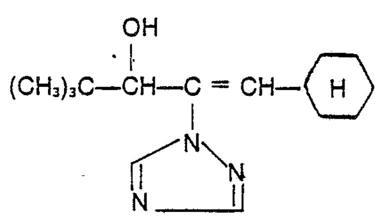

4. 1-Cyclohexyl-3-ethoxy-4,4-dimethyl-2-(1,2,4-triazol-1-yl)-pent-1-ene of the formula

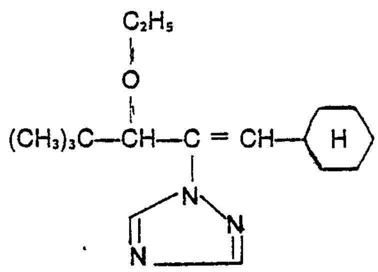

5. Process for the preparation of 1-vinyltriazoles of the formula

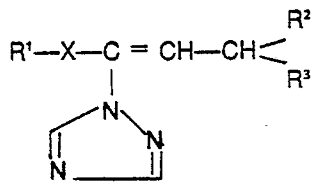 (I)

in which

$R^1$ represents alkyl with 1 to 4 carbon atoms which is optionally substituted by halogen, alkylcarbonyloxy with 1 to 4 carbon atoms in the alkyl part, alkylsuphonyloxy with 1 to 4 carbon atoms and/or phenylsuphonyloxy, which can be substituted by halogen and/or alkyl with 1 to 4 carbon atoms, cycloalkyl with 5 to 7 carbon atoms or aryl with 6 to 10 carbon atoms, it being possible for these aryl radicals to be substituted by halogen, alkyl with 1 to 4 carbon atoms, phenyl, phenoxy, halogenophenyl and/or halogenophenoxy, $R^2$ represents alkyl with 1 to 4 carbon atoms, $R^3$ represents alkyl with 1 to 4 carbon atoms, cycloalkyl with 5 to 7 carbon atoms, cycloalkenyl with 5 to 7 carbon atoms which is optionally substituted by alkyl with 1 to 4 carbon atoms, alkenyl with 2 to 4 carbon atoms or aryl with 6 to 10 carbon atoms which is optionally substituted hy halogen and/or alkyl with 1 to 4 carbon atoms, $R^2$ and $R^3$, together with the carbon atom to which they are bonded, also represent cycloalkenyl with 5 to 7 carbon atoms which is optionally substituted by alkyl with 1 to 4 carbon atoms or cycloalkyl with 3 to 7 carbon atoms, X represents the group -C($OR^4$)$R^5$ and additionally represents the keto group if $R^1$ represents optionally substituted alkyl with 1 to 4 carbon atoms or cycloalkyl, $R^4$ represents hydrogen, alkyl with 1 to 4 carbon atoms, aralkyl with 1 or 2 carbon atoms in the alkyl part and 6 to 10 carbon atoms in the aryl part which is optionally substituted by halogen, alkyl with 1 to 4 carbon atoms, halogenalkyl with up to 2 carbon atoms and up to 3 identical or different halogen atoms and/or phenyl or phenoxy which in their turn can be substituted by halogen, the acyl radical -CO-$R^{10}$ or the carbamoyl radical -CO-$NR^{11}$-$R^{12}$, $R^5$ represents hydrogen, alkyl with 1 to 4 carbon atoms or aralkyl with 1 or 2 carbon atoms in the alkyl part and 6 to 10 carbon atoms in the aryl part which is optionally substituted by halogen or alkyl with 1 to 4 carbon atoms, $R^{10}$ represents alkyl with 1 to 4 carbon atoms, halogenoalkyl with 1 or 2 carbon atoms and 1 to 5 identical or different halogen atoms or phenyl or benzyl which are optionally substituted by halogen and/or alkyl with 1 to 4 carbon atoms, $R^{11}$ represents hydrogen or alkyl with 1 to 4 carbon atoms and $R^{12}$ represents alkyl with 1 to 8 carbon atoms, halogenoalkyl with up to 4 carbon atoms and up to 5 identical or different halogen atoms, halogenoalkylmercapto with up to 2 carbon atoms and up to 5 halogen atoms or aryl with 6 to 10 carbon atoms which is optionally substituted by halogen, alkyl with 1 to 4 carbon atoms and/or halogenoalkyl with up to 2 carbon atoms and up to 5 identical or different halogen atoms, and acid addition salts and metal salt complexes thereof, characterised in that

a) triazole-ketones of the formula

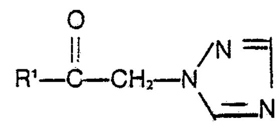 (II)

in which

$R^1$ has the meaning indicated above, are reacted with aldehydes of the formula

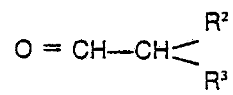 (III)

in which

$R^2$ and $R^3$ have the meaning indicated above, in the presence of a solvent and in the presence of a catalyst, and from the isomers which form, as a result of splitting off of water, the desired isomeric product of the formula

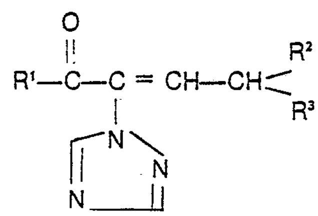 (Ia)

in which

$R^1$, $R^2$ and $R^3$ have the meaning indicated above, is isolated by customary methods, or

b) compounds obtainable by process a), of the formula

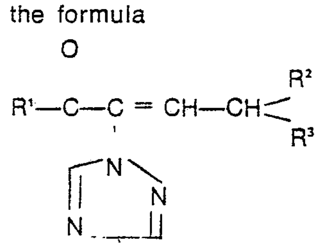 (Ia)

in which

$R^1$, $R^2$ and $R^3$ have the meaning indicated above, are either

α) reduced with complex hydrides in the presence of a solvent, or

β) reduced with Grignard compounds of the formula

$R^6$—Mg—Hal (IV)

in which

$R^6$ represents alkyl with 1 to 4 carbon atoms or aralkyl with 1 to 2 carbon atoms in the alkyl part and 6 to 10 carbon atoms in the aryl part which is optionally substituted by halogen or alkyl with 1 to 4 carbon atoms and

Hal represents chlorine, bromine or iodine, in the presence of a solvent, or

c) compounds obtainable by process (b), variants $\alpha$ and $\beta$, of the formula

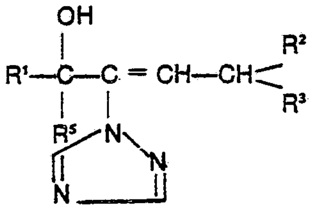

(Ib)

in which

$R^1$, $R^2$, $R^3$ and $R^5$ have the meaning indicated above, are reacted with halides of the formula

$R^7$—Hal' (V)

in which

$R^7$ represents alkyl with 1 to 4 carbon atoms, aralkyl with 1 to 2 carbon atoms in the alkyl part and 6 to 10 carbon atoms in the aryl part which is optionally monosubstituted or polysubstituted by identical or different substituents which are halogen, alkyl with 1 to 4 carbon atoms, halogenoalkyl with up to 2 carbon atoms and up to 3 halogen atoms, phenyl which is optionally substituted by halogen or phenoxy, or the radicals of the formulae -CO-$R^{10}$ and -CO-$NR^{11}R^{12}$ in which $R^{10}$, $R^{11}$ and $R^{12}$ have the meaning indicated above, and

Hal' represents fluorine, chlorine or bromine, in the presence of a solvent, and if appropriate in the presence of a strong base or if appropriate in the presence of an acid-binding agent, or

d) compounds obtainable by process (b) variants $\alpha$ and $\beta$, of the formula

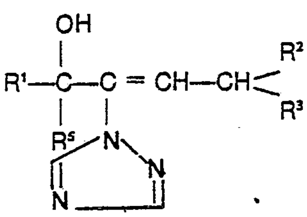

(Ib)

in which

$R^1$, $R^2$, $R^3$ and $R^5$ have the meaning indicated above, are reacted with acid anhydrides of the formula

$R^8$—O—$R^8$ (VI)

in which

$R^8$ represents the acyl radical of the formula -CO-$R^{10}$, wherein

$R^{10}$ has the meaning indicated above, in the presence of a solvent and if appropriate in the presence of a catalyst, or

e) compounds obtainable by process (b), variants $\alpha$ and $\beta$, of the formula

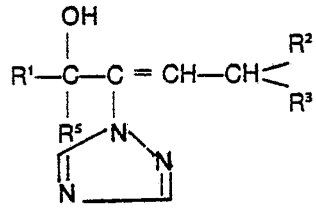

(Ib)

are reacted with isocyanates of the formula

O=C=N—$R^9$ (VII)

in which

$R^9$ represents alkyl with 1 to 8 carbon atoms, halogenoalkyl with up to 4 carbon atoms and up to 5 identical or different halogen atoms or aryl with 6 to 10 carbon atoms which is optionally monosubstituted or polysubstituted by identical or different substitutents which are halogen, alkyl with 1 to 4 carbon atoms, halogenoalkyl with up to 2 carbon atoms and up to 5 identical or different halogen atoms or halogenoalkylmercapto with 1 to 2 carbon atoms and up to 5 halogen atoms, in the presence of a solvent and if appropriate in the presence of a catalyst, and an acid or a metal salt is then optionally added onto the 1-vinyltriazole derivatives obtainable by the processes (a) to (e).

6. Plant growth regulating and fungicidal agents, characterised in that they contain at least one 1-vinyltriazole derivative of the formula (I) according to Claim 1 or an acid addition salt or metal salt complex of a 1-vinyltriazole derivative of the formula (I) according to Claim 1.

7. Use of 1-vinyltriazole derivatives of the formula (I) according to Claim 1 or of acid addition salts or metal salt complexes of 1-vinyltriazole derivatives of the formula (I) according to Claim 1, for regulating plant growth and for combating fungi.

8. Process for the preparation of plant growth regulating agents or of fungicidal agents, characterised in that 1-vinyltriazole derivatives of the formula (I) according to Claim 1 or acid addition salts or metal salt complexes of 1-vinyltriazole derivatives of the formula (I) according to Claim 1 are mixed with extenders and/or surface-active substances.

**Revendications**

1. Des 1-vinyltriazoles de formule:

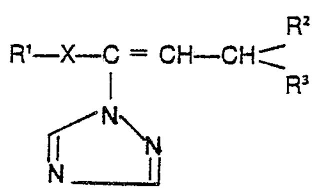

(I)

dans laquelle:

$R^1$ est un groupe alkyle ayant 1 à 4 atomes de carbone éventuellement substitué par un halogène, un groupe alkylcarbonyloxy ayant 1 à 4 atomes de carbone dans la partie alkyle, un groupe alkylsulfonyloxy ayant 1 à 4 atomes de

carbone et/ou un groupe phénylsulfonyloxy qui peut être substitué par un halogène et/ou un radical alkyle ayant 1 à 4 atomes de carbone, un groupe cycloalkyle ayant 5 à 7 atomes de carbone ainsi qu'un groupe aryle ayant 6 à 10 atomes de carbone, ces restes aryle pouvant être substitués par un halogène, un radical alkyle ayant 1 à 4 atomes de carbone, phényle, phénoxy, halogénophényle et/ou halogénophénoxy,

$R^2$ est un groupe alkyle ayant 1 à 4 atomes de carbone,

$R^3$ est un groupe alkyle ayant 1 à 4 atomes de carbone, cycloalkyle ayant 5 à 7 atomes de carbone, cycloalcényle ayant 5 à 7 atomes de carbone éventuellement substitué par un radical alkyle ayant 1 à 4 atomes de carbone, alcényle ayant 2 à 4 atomes de carbone ou un groupe aryle ayant 6 à 10 atomes de carbone éventuellement substitué par un halogène et/ou un radical alkyle ayant 1 à 4 atomes de carbone, $R^2$ et $R^3$ pouvant en outre former conjointement avec l'atome de carbone auquel ils sont liés, un groupe cycloalcényle ayant 5 à 7 atomes de carbone éventuellement substitué par un radical alkyle ayant 1 à 4 atomes de carbone ou un groupe cycloalkyle ayant 3 à 7 atomes de carbone,

X représente le groupe -C($OR^4$)$R^5$- ainsi que, en outre, le groupe céto, lorsque $R^1$ est un groupe alkyle ayant 1 à 4 atomes de carbone éventuellement substitué ou un grope cycloalkyle,

$R^4$ est l'hydrogène, un groupe alkyle ayant 1 à 4 atomes de carbone, un groupe aralkyle ayant 1 ou 2 atomes de carbone dans la partie alkyle, éventuellement substitué par un halogène, un radical alkyle ayant 1 à 4 atomes de carbone, un radical halogénalkyle ayant jusqu'à 2 atomes de carbone et jusqu'à 3 atomes d'halogène égaux ou différents et/ou un groupe phényle ou un groupe phénoxy, qui peuvent être substitués quant à eux par un halogène, le reste acyle -CO-$R^{10}$ ou le reste carbamoyle -CO-$NR^{11}R^{12}$,

$R^5$ est l'hydrogène, un groupe alkyle ayant 1 à 4 atomes de carbone ainsi qu'un groupe aralkyle ayant 1 ou 2 atomes de carbone dans la partie alkyle et 6 à 10 atomes de carbone dans la partie aryle, éventuellement substitué par un halogène ou par un radical alkyle ayant 1 à 4 atomes de carbone,

$R^{10}$ est un groupe alkyle ayant 1 à 4 atomes de carbone, un groupe halogénalkyle ayant 1 ou 2 atomes de carbone et 1 à 5 atomes d'halogènes égaux ou différents ainsi qu'un groupe phényle ou benzyle éventuellement substitué par un halogène et/ou un radical alkyle ayant 1 à 4 atomes de carbone,

$R^{11}$ est l'hydrogène ou un groupe alkyle ayant 1 à 4 atomes de carbone et

$R^{12}$ est un groupe alkyle ayant 1 à 8 atomes de carbone, un groupe halogénalkyle ayant jusqu'à 4 atomes de carbone et jusqu'à 5 atomes d'halogènes égaux ou différents, un groupe halogénalkylmercapto ayant jusqu'à 2 atomes de carbone et jusqu'à 5 atomes d'halogènes ainsi qu'un groupe aryle ayant 6 à 10 atomes de carbone éventuellement substitué par un halogène, un radical alkyle ayant 1 à 4 atomes de carbone et/ou un radical halogénalkyle ayant jusqu'à 2 atomes de carbone et jusqu'à 5 atomes d'halogènes égaux ou différents, ainsi que leurs sels d'addition d'acides et leurs complexes de sels métalliques.

2. La 1-cyclohexyl-4,4-diméthyl-2-(1,2,4-triazol-1-yl)-pent-1-ène-3-one de formule:

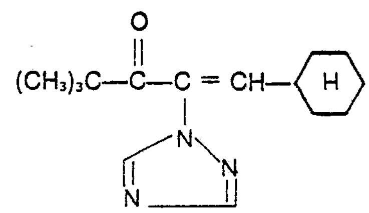

3. Le 1-cyclohexyl-4,4-diméthyl-2-(1,2,4-triazol-1-yl)-pent-1-ène-3-ol de formule:

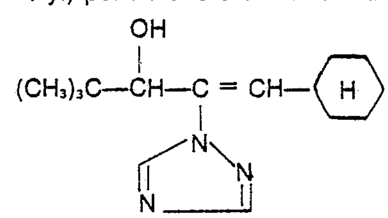

4. Le 1-cyclohexyl-3-éthoxy-4,4-diméthyl-2-(1,2,4-triazol-1-yl)-pent-1-ène de formule:

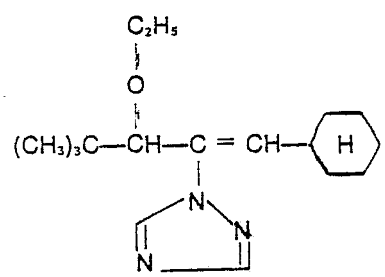

5. Procédé de production de 1-vinyltriazoles de formule:

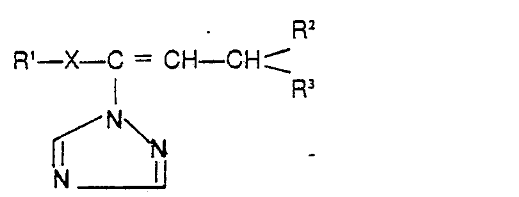 (I)

dans laquelle:

$R^1$ est un groupe alkyle ayant 1 à 4 atomes de carbone éventuellement substitué par un halogène, un groupe alkylcarbonyloxy ayant 1 à 4 atomes de carbone dans la partie alkyle, un groupe alkylsulfonyloxy ayant 1 à 4 atomes de carbone et/ou un groupe phénylsulfonyloxy qui peut être substitué par un halogène et/ou un radical alkyle ayant 1 à 4 atomes de carbone, un groupe cycloalkyle ayant 5 à 7 atomes de carbone ainsi qu'un groupe aryle ayant 6 à 10 atomes de carbone, ces restes aryle pouvant être substitués par un halogène, un radioal alkyle ayant 1 à 4 atomes de carbone, phényle, phénoxy, halogénophényl et/ou halogénophén-

oxy, $R^2$ est un groupe alkyle ayant 1 à 4 atomes de carbone, $R^3$ est un groupe alkyle ayant 1 à 4 atomes de carbone, cycloalkyle ayant 5 à 7 atomes de carbone, cycloalcényle ayant 5 à 7 atomes de carbone éventuellement substitué par un radical alkyle ayant 1 à 4 atomes de carbone, alcényle ayant 2 à 4 atomes de carbone ou un groupe aryle ayant 6 à 10 atomes de carbone éventuellement substitué par un halogène et/ou un radical alkyle ayant 1 à 4 atomes de carbone, $R^2$ et $R^3$ pouvant en outre former conjointement avec l'atome de carbone auquel ils sont liés, un groupe cycloalcényle ayant 5 à 7 atomes de carbone éventuellement substitué par un radical alkyle ayant 1 à 4 atomes de carbone ou un groupe cycloalkyle ayant 3 à 7 atomes de carbone, X représente le groupe -C(OR⁴)-R⁵- ainsi que, en outre, le groupe céto, lorsque $R^1$ est un groupe alkyle ayant 1 à 4 atomes de carbone éventuellement substitué ou un groupe cycloalkyle, $R^4$ est l'hydrogène, un groupe alkyle ayant 1 à 4 atomes de carbone, un groupe aralkyle ayant 1 ou 2 atomes de carbone dans la partie alkyle et 6 à 10 atomes de carbone dans la partie aryle, éventuellement substitué par un halogène, un radical alkyle ayant 1 à 4 atomes de carbone, un radical halogénalkyle ayant jusqu'à 2 atomes de carbone et jusqu'à 3 atomes d'halogènes égaux ou différents et/ou un groupe phényle ou un groupe phénoxy, qui peuvent être substitués quant à eux par un halogène, le reste acyle $-CO-R^{10}$ ou le reste carbamoyle $-CO-NR^{11}R^{12}$, $R^5$ est l'hydrogène, un groupe alkyle ayant 1 à 4 atomes de carbone ainsi qu'un groupe aralkyle ayant 1 ou 2 atomes de carbone dans la partie alkyle et 6 à 10 atomes de carbone dans la partie aryle, éventuellement substitué par un halogène ou par un radical alkyle ayant 1 à 4 atomes de carbone, $R^{10}$ est un groupe alkyle ayant 1 à 4 atomes de carbone, un groupe halogénalkyle ayant 1 ou 2 atomes de carbone et 1 à 5 atomes d'halogènes égaux ou différents ainsi qu'un groupe phényle ou benzyle éventuellement substitué par un halogène et/ou un radical alkyle ayant 1 à 4 atomes de carbone, $R^{11}$ est l'hydrogène ou un groupe alkyle ayant 1 à 4 atomes de carbone et $R^{12}$ est un groupe alkyle ayant 1 à 8 atomes de carbone, un groupe halogénalkyle ayant jusqu'à 4 atomes de carbone et jusqu'à 5 atomes d'halogènes égaux ou différents, un groupe halogénalkylmercapto ayant jusqu'à 2 atomes de carbone et jusqu'à 5 atomes d'halogènes ainsi qu'un groupe aryle ayant 6 à 10 atomes de carbone éventuellement substitué par un halogène, un radical alkyle ayant 1 à 4 atomes de carbone et/ou un radical halogénalkyle ayant jusqu'à 2 atomes de carbone et jusqu'à 5 atomes d'halogènes égaux ou différents, ainsi que de leurs sels d'addition d'acides et de leurs complexes métalliques, caractérisé en ce que

a) on fait réagir des triazole-cétones de formule:

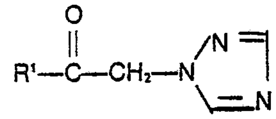
(II)

dans laquelle:

$R^1$ a la définition indiquée ci-dessus, avec des aldéhydes de formule:

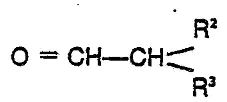
(III)

dans laquelle:

$R^2$ et $R^3$ ont la définition indiquée ci-dessus, en présence d'un solvant et en présence d'un catalyseur et on isole par des opérations classiques des isomères qui se forment par suite de l'élimination d'eau, le produit isomère de formule:

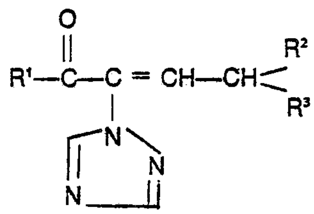
(Ia)

dans laquelle:

$R^1$, $R^2$ et $R^3$ ont la définition indiquée ci-dessus, ou

b) des composés pouvant être obtenus selon le procédé a), de formule:

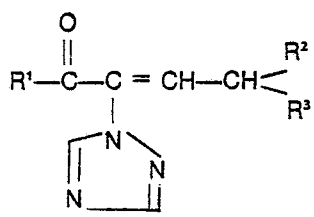
(Ia)

dans laquelle:

$R^1$, $R^2$ et $R^3$ ont la définition indiquée ci-dessus,

α) sont réduits avec des hydrures complexes en présence d'un solvant

β) sont réduits avec des composés de Grignard de formule:

$R^6$—Mg—Hal (IV)

dans laquelle:

$R^6$ est un groupe alkyle ayant 1 à 4 atomes de carbone ou un groupe aralkyle ayant 1 ou 2 atomes de carbone dans la partie alkyle et 6 à 10 atomes de carbone dans la partie aryle, éventuellement substitué par un halogène ou par un radical alkyle ayant 1 à 4 atomes de carbone et

Hal désigne du chlore, du brome ou de l'iode, en présence d'un solvant ou

c) des composés pouvant être obtenus selon les variantes α et β du procédé (b), de formule:

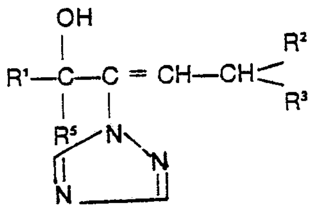

(Ib)

dans laquelle:

$R^1$, $R^2$, $R^3$ et $R^5$ ont la définition donnée ci-dessus, sont amenés à réagir avec des halogénures de formule:

$R^7$--Hal' (V)

dans laquelle:

$R^7$ est un groupe alkyle ayant 1 à 4 atomes de carbone, un groupe aralkyle ayant 1 ou 2 atomes de carbone dans la partie alkyle et 6 à 10 atomes de carbone dans la partie aryle, portant éventuellement un ou plusieurs substituants égaux ou différents tels qu'halogéno, alkyle ayant 1 à 4 atomes de carbone, halogénalkyle ayant jusqu'à 2 atomes de carbone et jusqu'à 3 atomes d'halogènes, phényle éventuellement substitué par un halogène ou phénoxy, ou les restes de formules -CO-$R^{10}$ et -CO-$NR^{11}R^{12}$ dans lesquelles $R^{10}$, $R^{11}$ et $R^{12}$ ont la définition indiquée ci-dessus, et

Hal' désigne du fluor, du chlore ou du brome, en présence d'un solvant et, le cas échéant en présence d'une base forte ou éventuellement en présence d'un accepteur d'acide, ou

d) des composés pouvant être obtenus selon les variantes $\alpha$ et $\beta$ du procédé (b), de formule:

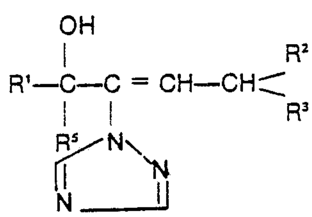

(Ib)

dans laquelle:

$R^1$, $R^2$, $R^3$ et $R^5$ ont la définition indiquée ci-dessus, sont amenés à réagir avec des anhydrides d'acides de formule:

$R^8$—O—$R^8$ (VI)

dans laquelle:

$R^8$ est le reste acyle de formule -CO-$R^{10}$ où

$R^{10}$ a la définition indiquée ci-dessus, en présence d'un solvant et, le cas échéant en présence d'un catalyseur, ou

e) des composés pouvant être obtenus selon les variantes $\alpha$ et $\beta$ du procédé (b), de formule:

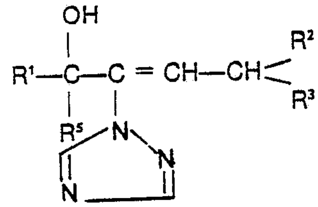

(Ib)

sont amenés à réagir avec des isocyanates de formule:

O=C=N—$R^9$ (VII)

dans laquelle:

$R^9$ est un groupe alkyle ayant 1 à 8 atomes de carbone, halogénalkyle ayant jusqu'à 4 atomes de carbone et jusqu'à 5 atomes d'halogènes égaux ou différents ou un groupe aryle ayant 6 à 10 atomes de carbone portant éventuellement un ou plusieurs substituants égaux ou différents tels qu'halogéno, alkyle ayant 1 à 4 atomes de carbone, halogénalkyle ayant jusqu'à 2 atomes de carbone et jusqu'à 5 atomes d'halogènes égaux ou différents ou halogénalkylmercapto ayant 1 ou 2 atomes de carbone et jusqu'à 5 atomes d'halogènes, en présence d'un solvant et, le cas échéant en présence d'un catalyseur, puis un acide ou un sel métallique est éventuellement additionné ensuite sur les dérivés 1-vinyltriazoliques obtenus par les procédés (a) à (e).

6. Compositions régulatrices de croissance des plantes et fongicides, caractérisée par une teneur en au moins un dérivé 1-vinyltriazolique de formule (I) suivant la revendication 1 ou un sel d'addition d'acide ou un complexe de sel métallique d'un dérivé 1-vinyltriazolique de formule (I) suivant la revendication 1.

7. Utilisation de dérivés 1-vinyltriazoliques de formule (I) suivant la revendication 1 ou de sels d'addition d'acides ou de complexes de sels métalliques de dérivés 1-vinyltriazoliques de formule (I) suivant la revendication 1 pour la régulation de la croissance des plantes ou pour la lutte contre des champignons.

8. Procédé de production de compositions régulatrices de croissance des plantes et d'agents fongicides, caractérisé en ce qu'on mélange des dérivés 1-vinyltrazoliques de formule (I) suivant la revendication 1 ou des sels d'addition d'acides ou des complexes de sels métalliques de dérivés 1-vinyltriazoliques de formule (I) suivant la revendication 1 avec des diluants et/ou des agents tensio-actifs.